# EUROPEAN PATENT APPLICATION

(11) **EP 4 811 376 A1**
(43) Date of publication of application: **23.09.2026**
(21) Application number: 26165480.0
(22) Date of filing: 17.03.2026
(51) Int. Cl.: G16H 20/40

(54) **DYNAMIC ADJUSTMENT OF RADIOTHERAPY GRAPHICAL USER INTERFACES**

(30) Priority: 21.03.2025 US 202519087296
(71) Applicant: Varian Medical Systems, Inc., Palo Alto, CA 94304 (US)
(72) Inventor: SPATOLA, Brian, Palo Alto, 94304 (US); VOJAN, Filip, Palo Alto, 94304 (US)
(74) Representative: Mathisen & Macara LLP

(57) **Abstract**

A display screen (226) on a gantry (224) of a radiotherapy machine (241) presents a GUI having a set of icons (308, 310) to assist an operator with guidance for setup and adjustments. During rotation of the gantry (224), the set of icons (308, 310) are dynamically adjusted on a display screen (226). During the dynamic adjustment, the set of icons (308, 310) are relocated on the display screen (226) such that they maintain an orientation relative to a reference (e.g., a couch (222) within the radiotherapy system (240)) despite the rotation of the display screen (226). In some implementations, the dynamic adjustment includes removing the set of icons (308, 310) from a first position and displaying them at a second position during rotation of the gantry (224) to avoid obfuscating the set of icons (308, 310) behind a component of the radiotherapy system (240). As a result, the operator may continue to interact with the GUI in an efficient manner even though the display screen (226) may be rotated from its original and upright position.

## Description

### TECHNICAL FIELD

This application relates generally to graphical user interfaces and software for radiotherapy treatment setup and machines.

### BACKGROUND

Radiation therapy, which utilizes ionizing radiation, is a localized treatment targeting specific tissues, such as cancerous tumors. Ideally, this therapy focuses on the planning target volume (PTV) or target organ, sparing surrounding healthy tissue from excessive radiation doses to minimize damage. To ensure the prescribed dose is accurately delivered to the PTV, the patient must be precisely positioned relative to the linear accelerator, typically using a movable treatment couch on a turntable assembly. Implementing radiation therapy is a complex process involving specific guidelines, protocols, and instructions followed by various medical professionals, including clinicians, technicians, device manufacturers, and treating physicians. Due to the hazards associated with radiation, it is crucial that all instructions are meticulously followed.

Conventional radiation therapy systems are often complex to operate, requiring extensive training for efficient workflow execution. Tasks such as patient positioning, system calibration, and machine adjustments involve numerous interactions with both the patient and the radiotherapy machine. The data needed for these setups is often voluminous and complex. However, these conventional methods can be inefficient, ineffective, or even dangerous.

Conventional radiotherapy systems do not efficiently display the data needed for patient and machine setup. In some conventional systems and methods, the necessary information for an operator (e.g., radiotherapy technician or any other medical professional) is not presented within the treatment room or in a manner that is intuitive and user-friendly, interrupting the adjustment process and proving to be ineffective and time-consuming. Some other conventional systems and methods provide all the information via static and complex graphical user interfaces (GUIs). Unless an operator is highly experienced with a particular system, this can slow down the setup process and degrade the patient's experience.

### SUMMARY

In accordance with a first aspect of the invention, there is provided a computer-readable medium as defined by claim 1.

In accordance with a second aspect of the invention, there is provided a system as defined by claim 9.

In accordance with a third aspect of the invention, there is provided a method as defined by claim 14.

Optional features are defined by the dependent claims.

Given the complexities involved and discussed herein, there is a need for an intuitive and workflow-oriented graphical user interface (GUI) specific to radiotherapy treatments that efficiently displays treatment setup data, such as machine parameters, patient alignment data, and necessary accessories. As described herein, a display screen on a gantry of a radiotherapy machine presents a GUI having a set of icons to assist an operator with guidance for setup and adjustments. During rotation of the gantry, the set of icons are dynamically adjusted on a display screen. During the dynamic adjustment, the set of icons are relocated on the display screen such that they maintain an orientation relative to a reference (e.g., a couch within the radiotherapy system) despite the rotation of the display screen. In some implementations, the dynamic adjustment includes removing the set of icons from a first position and displaying them at a second position during rotation of the gantry to avoid obfuscating the set of icons behind a component of the radiotherapy system. As a result, the operator may continue to interact with the GUI in an efficient manner even though the display screen may be rotated from its original and upright position.

In one embodiment, the techniques described herein relate to a computer-readable medium with instructions stored therein that when executed by one or more processors, cause the one or more processors to: present on a display screen located on a gantry of a radiotherapy machine, a set of icons corresponding to one or more attributes of a radiotherapy treatment implemented via the radiotherapy machine; receive a first indication of a movement of the gantry of the radiotherapy machine; and upon receiving the first indication of the movement of the gantry of the radiotherapy machine, relocate a subset of the set of icons on the display screen, such that the subset of the set of icons appears to be not rotating relative to a movement of the gantry of the radiotherapy machine.

The instructions may cause the one or more processors, upon receiving the first indication of the movement of the gantry of the radiotherapy machine, relocate a second subset of the set of icons on the display screen, such that the second subset of the set of icons appear to be rotating consistent with the movement of the gantry.

The instructions may cause the one or more processors to: remove the second subset of the set of icons from a first position when the gantry has rotated beyond a first defined position; and display at a second position the second subset of the set of icons previously removed when the gantry has rotated beyond a second defined position.

The second subset of the set of icons may display an identifier of a patient being treated.

The instructions may cause the one or more processors to: disable the display screen when the gantry is at a defined position.

The instructions may cause the one or more processors to: obfuscate at least one icon within the set of icons when the gantry reaches a defined position.

Relocating of the second subset of the set of icons may be consistent with a movement parameter of the movement of the gantry of the radiotherapy machine.

The instructions may cause the one or more processors to: receive a second indication of the movement parameter of the gantry of the radiotherapy machine; determine an icon movement parameter for the second subset of the set of icons, wherein the icon movement parameter corresponds to the movement parameter of the gantry; and upon receiving the first indication of the movement of the gantry of the radiotherapy machine, relocate the second subset of the set of icons on the display screen in accordance with the icon movement parameter, such that the second subset of the set of icons appear to be rotating with the movement of the gantry.

The movement parameter of the gantry of the radiotherapy machine may include at least one of a speed of rotation of the movement of the gantry, a direction of rotation of the movement of the gantry, an acceleration of the movement of the gantry, or an angle of rotation of the movement of the gantry.

Relocating the second subset of the set of icons on the display screen, such that the second subset of the set of icons appear to be rotating consistent with the movement of the gantry may further include: adjusting the second subset of the set of icons with respect to the display screen at a first angle of rotation opposite of a second angle of rotation of the movement of the gantry.

The instructions may cause the one or more processors to: maintain a location of the second subset of the set of icons relative to the display screen such that the second subset of the set of icons rotate about a datum point on the display screen and wherein the second subset of the set of icons are further adjusted at the first angle of rotation about a secondary datum point.

In another embodiment, the techniques described herein relate to a system including: a radiotherapy machine including a gantry with a display screen located thereon; one or more processors; and a computer-readable, non-transitory medium with instructions stored therein that when executed by the one or more processors, cause the one or more processors to: present on the display screen located on the gantry of the radiotherapy machine, a set of icons corresponding to one or more attributes of a radiotherapy treatment implemented via the radiotherapy machine; receive a first indication of a movement of the gantry of the radiotherapy machine; and upon receiving the first indication of the movement of the gantry of the radiotherapy machine, relocate a subset of the set of icons on the display screen, such that the subset of the set of icons appear to be not rotating relative to the movement of the gantry of the radiotherapy machine.

The instructions may further cause the one or more processors to, upon receiving the first indication of the movement of the gantry of the radiotherapy machine, relocate a second subset of the set of icons on the display screen, such that the second subset of the set of icons appear to be rotating consistent with the movement of the gantry.

The instructions may further cause the one or more processors to: remove the second subset of the set of icons from a first position when the gantry has rotated beyond a first defined position; and display at a second position the second subset of the set of icons previously removed when the gantry has rotated beyond a second defined position.

Relocating of the second subset of the set of icons may correspond with a movement parameter of the movement of the gantry of the radiotherapy machine.

The instructions may further cause the one or more processors to: receive a second indication of the movement parameter of the gantry of the radiotherapy machine; determine an icon movement parameter for the second subset of the set of icons, wherein the icon movement parameter corresponds to the movement parameter of the gantry; and upon receiving the first indication of the movement of the gantry of the radiotherapy machine, relocate the second subset of the set of icons on the display screen in accordance with the icon movement parameter, such that the second subset of the set of icons appear to be rotating with the movement of the gantry while maintaining a first orientation.

The instructions may further cause the one or more processors to adjust a first orientation of the second subset of the set of icons with respect to the display screen at a first angle of rotation opposite of a second angle of rotation of the movement of the gantry such that a second orientation of the second subset of the set of icons is maintained when relocating the second subset of the set of icons on the display screen, such that the second subset of the set of icons appears to be rotating consistent with the movement of the gantry.

The instructions further cause the one or more processors to maintain a location of the second subset of the set of icons relative to the display screen such that the second subset of the set of icons rotate about a datum point on the display screen.

In yet another embodiment, the techniques described herein relate to a method including: presenting, by at least one processor on a display screen located on a gantry of a radiotherapy machine, a set of icons corresponding to one or more attributes of a radiotherapy treatment implemented via the radiotherapy machine; receiving, by the at least one processor, a first indication of a movement of the gantry of the radiotherapy machine; and upon receiving the first indication of the movement of the gantry of the radiotherapy machine, relocating, by the at least one processor, a subset of the set of icons on the display screen, such that the subset of the set of icons appear to be not rotating relative to the movement of the gantry of the radiotherapy machine.

The method may further include: upon receiving the first indication of the movement of the gantry of the radiotherapy machine, relocating, by the at least one processor, a second subset of the set of icons on the display screen, such that the second subset of the set of icons appear to be rotating consistent with the movement of the gantry.

The methods discussed herein may assist the user in performing a radiotherapy setup workflow, by means of a continued and/or guided process of human-machine interaction. The systems discussed herein may be configured to assist the user in performing a radiotherapy workflow and/or a radiotherapy setup workflow, by means of a continued and/or guided process of human-machine interaction.

### BRIEF DESCRIPTION OF THE DRAWINGS

Non-limiting embodiments of the present disclosure are described by way of example with reference to the accompanying figures, which are schematic and are not intended to be drawn to scale. Unless indicated as representing the background art, the figures represent aspects of the disclosure.
**FIG. 1** illustrates components of a workflow-oriented radiotherapy system, according to an embodiment.
**FIG. 2A** illustrates a radiotherapy machine in a default, upright orientation, according to an embodiment.
**FIG. 2B** illustrates a radiotherapy machine in a rotated orientation, according to an embodiment.
**FIG. 3A** illustrates a radiotherapy machine in a default, upright orientation and displaying a set of icons, according to an embodiment.
**FIG. 3B** illustrates a radiotherapy machine in a rotated orientation and displaying a set of icons, according to an embodiment.
**FIG. 3C** illustrates a radiotherapy machine in a rotated orientation and displaying a set of icons, according to an embodiment.
**FIG. 3D** illustrates a radiotherapy machine in a rotated orientation and displaying a set of icons, according to an embodiment.
**FIG. 4A** illustrates a radiotherapy machine in a default, upright orientation and displaying a set of icons, according to an embodiment.
**FIG. 4B** illustrates a radiotherapy machine in a rotated orientation and displaying a set of icons, according to an embodiment.
**FIG. 4C** illustrates a radiotherapy machine in a rotated orientation and displaying a set of icons, according to an embodiment.
**FIG. 4D** illustrates a radiotherapy machine in a rotated orientation and displaying a set of icons, according to an embodiment.
**FIG. 5** is a flow diagram of a method for dynamically adjusting a position and orientation of a set of icons on a radiotherapy machine during rotation of the radiotherapy machine, according to an embodiment.

### DETAILED DESCRIPTION

Reference will now be made to some embodiments illustrated in the drawings, and specific language will be used here to describe the same. It will nevertheless be understood that no limitation of the scope of the embodiments of the methods and systems described herein is thereby intended. Alterations and further modifications of the features illustrated here, and additional applications of the principles of the embodiments of the methods and systems described herein, as illustrated here, which would occur to a person skilled in the relevant art and having possession of this disclosure, are to be considered within the scope of the embodiments, methods, and/or systems described herein.

Certain radiotherapy machines used during administration of radiotherapy treatments include screens mounted to the radiotherapy machine to aid in the administration of the radiotherapy treatment. These screens may be mounted to a gantry of the machine, which is able to rotate around a patient to be treated during set up and delivery of the radiotherapy. The screen displays a GUI having multiple icons that provide details related to the radiotherapy process, such as setup parameters/adjustments, real-time positioning information, and/or patient information. As the gantry of the radiotherapy machine rotates, the screen may counter-rotate to allow for a consistent orientation of the GUI when the gantry and the screen of the gantry have changed position.

To maintain legibility of the icons during rotation of the gantry, positions of the icons within both categories of icons are dynamically adjusted on the GUI during the rotation of the gantry. The first category of icons may be located at a periphery of the display screen. The icons in the first category of icons are dynamically adjusted during gantry rotation so that they appear to rotate as the gantry rotates but maintains an upright orientation. The icons in the second category of icons are also dynamically adjusted during gantry rotation so that they appear to be stationary, irrespective of the gantry's rotation.

### System Architecture

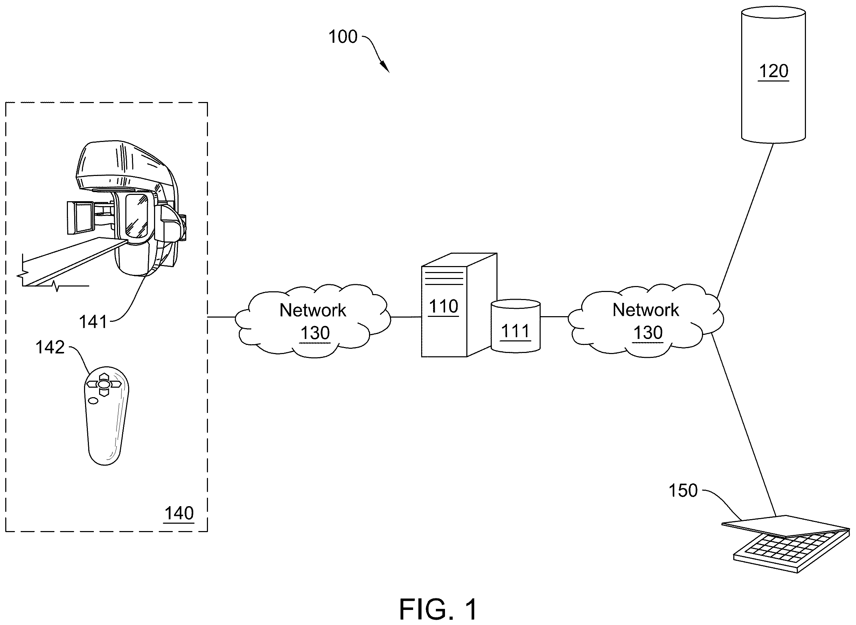

The methods described herein can be implemented using various computing devices/features described in **FIG. 1****.** Therefore, **FIG. 1** describes a non-limiting example of a computer environment where a server can perform the processes/methods described herein, such as retrieving, processing, and presenting radiotherapy treatment data, and presenting data for a graphical user interface (GUI) having workflow-oriented pages (or instances) on various displays screens.

**FIG. 1** illustrates various components of a system **100** for presenting various pages related to operation of a radiotherapy treatment, in accordance with an embodiment. The system 100 may include an analytics server **110,** a medical records database **120,** a radiotherapy system **140,** and a system administrator computer **150** or workstation. These features may communicate with each other over a network **130.** For example, the system **100** may present, optionally via the analytics server **110,** one or more pages/GUIs on the radiotherapy machine **141.** In another example, the system **100** may present, optionally via the analytics server **110,** a page presenting a live feed of a patient on one or more display devices, such as the display device on a gantry of the radiotherapy machine **141.** In general, any of the steps discussed herein as performed by the analytics server may be performed by the system in general.

The network **130** may include wired and/or wireless communications according to one or more standards via one or more transport mediums. Communication over the network **130** may be in accordance with various communication protocols, such as transmission control protocol and internet protocol (TCP/IP), user datagram protocol (UDP), and Institute of Electrical and Electronics Engineers (IEEE) communication protocols. The network **130** may further include wireless communications according to Bluetooth specification sets, or another standard or proprietary wireless communication protocol. The network **130** may further include communications over a cellular network, including, for example, a global system for mobile (GSM) communications, code division multiple access (CDMA), and enhanced data for global evolution network (EDGE). The examples of the network 130 may include, but are not limited to, private or public local area network (LAN), wireless LAN (WLAN), metropolitan area network (MAN), wide area network (WAN), and the Internet.

The analytics server **110** may be any computing device capable of performing the actions described herein. For instance, the analytics server **110** includes a processing unit and a non-transitory machine-readable storage medium. The processing unit includes a processor with a computer-readable medium, such as a random-access memory coupled to the processor. In some embodiments, the analytics server **110** executes algorithms or computer executable program instructions, which may be executed by a single processor or multiple processors in a distributed configuration. The instructions allow the processor to implement the functionality described herein. The analytics server **110** may be configured to interact with one or more software modules of a same or a different type operating within the system **100.**

Non-limiting examples of the processor may include a microprocessor, an application specific integrated circuit, and a field programmable object array, among others. The analytics server **110** may be capable of executing data processing tasks, data analysis tasks, and valuation tasks. Non-limiting examples of the analytics server **110** may include a desktop computer, a server computer, a laptop computer, a tablet computer, and the like. For simplicity, **FIG. 1** depicts a single-server computing device functioning as the analytics server **110.** However, some embodiments may include a plurality of server computing devices capable of performing various tasks described herein.

Implementation of the methods described herein is not limited to the system architecture depicted in **FIG. 1****.** In alternative embodiments, the analytics server **110** may be an embedded computing device disposed within the radiotherapy machine **141.** In some embodiments, the analytics server **110** may be a plurality of computing devices operated locally and/or remotely. In various embodiments, the analytics server **110** may be operated through a cloud service (e.g., network, internet). The cloud service may be performed in accordance with various communication protocols such as TCP/IP, UDP, and IEEE communication protocols. The analytics server **110** may utilize a database, such as a local database **111,** to store and/or retrieve various data described herein. For instance, the analytics server **110** may store different data corresponding to the different pages within the local database **111.** The page may be displayed on a display screen associated with the radiotherapy system **140.**

For instance, the analytics server **110** may display a page having a live feed of the patient before the treatment has begun and/or may display various pages describing how to position the patient on the couch and/or how to adjust the radiotherapy machine **141.** Even though some embodiments describe the analytics server **110** displaying various pages on a display screen attached to the radiotherapy machine **141** (e.g., located on the gantry of the radiotherapy machine **141),** it is expressly understood that the analytics server **110** may display different pages described herein on a display screen located anywhere within the treatment room, such as located on the wall of the treatment room or on any electronic device within the treatment room. In some configurations, the analytics server **110** may display the pages on the system administrator computer **150** or workstation.

The local database **111** may also store data corresponding to the radiotherapy machine **141** (e.g., default position of the radiotherapy machine **141,** and/or current position of the radiotherapy machine **141,** such as the orientation of the bed/couch and/or gantry).

If the analytics server **110** receives a request from the radiotherapy system **140** to provide the current position/orientation of the radiotherapy machine **141,** the analytics server **110** may query the local database **111** and may retrieve the corresponding data. Additionally, or alternatively, the analytics server **110** may also communicate with various sensors associated with the radiotherapy system **140** to retrieve machine data and parameters. The analytics server **110** may then use the methods/systems described herein to instruct the radiotherapy machine **141** to adjust the positioning of the radiotherapy machine **141** to the default position of the radiotherapy machine **141,** or another position of the radiotherapy machine, or to display one or more pages instructing a technician to adjust the radiotherapy machine **141** and/or the patient.

The local database **111** may also store data associated with different users of the radiotherapy system **140.** In a non-limiting example, the user data may include the user's login (e.g., sign-in, credentials) information and authorization levels. For example, the analytics server **110** may store, using the local database **111,** the user's name and password. The analytics server **110** may allow the user to log in to the radiotherapy machine **141,** and/or the system administrator computer **150.** If the user logs-in into the system, the analytics server **110** may adjust what the user is able to view, adjust, and control based on the user's authorization level. The analytics server **110** may conceal the patient's medical information that is not relevant to the radiation treatment from the medical technician. In another example, the analytics server **110** may conceal all of the patient's medical information if a machine technician signs-in.

The analytics server **110** may also utilize one or more other databases, such as the medical records database **120,** to store and/or retrieve various data described herein. The databases herein can be configured as one or more databases storing the data, and the disclosure is not intended to be limited to a particular number or location of databases. The analytics server **110** may instruct the medical records database **120** to store patient data (e.g., patient name, patient machine alignment information) associated with a patient identifier (e.g., patient's name, patient's profile image). The analytics server **110** may then instruct the medical records database **120** to populate a dataset corresponding to a patient and display the profile image of the patient. If the analytics server **110** receives a request from the radiotherapy system **140** to display the data associated with the patient identifier, the analytics server **110** may query the medical records database **120** and may retrieve the corresponding dataset. The analytics server **110** may then use the methods/systems described herein to dynamically display the patient data in accordance with the patient identifier.

The medical records database **120** may also include a radiotherapy treatment file associated with the patient identifier. As used herein, the radiotherapy treatment file refers to all data associated with a patient's radiation therapy treatment and is not limited to a particular step or information. The radiotherapy treatment file may also be retrieved from the radiotherapy system **140,** the medical records database **120,** and/or the system administrator computer **150.** The radiotherapy treatment file may include the treatment data associated with a patient. The radiotherapy treatment file may be associated with a patient identifier and may also be updated after a patient has completed treatment. For example, after the patient has completed a treatment session, the medical records database **120** may retrieve the duration of the treatment session from the radiotherapy system **140** and update the radiotherapy file to include the duration of the treatment session. Even though aspects of the embodiments described herein discuss radiotherapy treatment as a file, the radiotherapy treatment file represents a collection of the patient's medical and treatment data, which may be stored in different files. For brevity, the present disclosure refers to the patient's data as a radiotherapy treatment file.

The radiotherapy treatment file may include data for treatment plans for one or more patients where each treatment plan is specific to a single patient. For instance, each treatment plan is uniquely created for each patient and corresponds to the patient's unique attributes (e.g., physical attributes of the patient and the patient's unique condition to be treated). In some configurations, each treatment plan for each patient may itself include multiple files.

The analytics server **110** may retrieve treatment data associated with a patient that is stored within the patient's radiotherapy treatment file(s). As described herein, the analytics server **110** may analyze the treatment data and may display various features and graphical components described herein. While the medical records database **120** may contain treatment data (radiotherapy treatment files) associated with multiple patients, the methods described herein are implemented, such that various pages and graphical features described herein are specific to the particular patient being treated.

In some configurations, the analytics server **110** may retrieve radiotherapy treatment files associated with multiple patients. The analytics server **110** may then receive a selection by an operator (e.g., technician) of a patient to be treated. As a result, the analytics server **110** identifies the radiotherapy treatment file associated with the selected patient and customizes the graphical components described herein for the selected patient.

The analytics server **110** may also retrieve and instruct the medical records database **120** to store patient data associated with the patient from the medical records database **120,** the radiotherapy system **140,** and/or the system administrator computer **150.** For instance, the medical records database **120** may include patient data (e.g., previously populated by the analytics server **110** and/or periodically retrieved from a third-party data source). If a patient is selected, the analytics server **110** may query and retrieve patient data from the medical records database **120** and provide the retrieved patient data. For instance, the analytics server **110** may display the patient's profile image when the patient is selected, providing an opportunity to verify the correct patient was selected.

The analytics server **110** may receive treatment data associated with a patient from the medical records database **120,** the treatment data may further include at least a patient identifier. The analytics server may receive radiotherapy treatment file associated with one or more patients from the medical records database **120.** The radiotherapy treatment file may refer to a file having data associated with a process in which a medical team (e.g., radiation oncologists, radiation therapist, medical physicists, and/or medical dosimetrists) plan the appropriate external beam radiotherapy or internal brachytherapy treatment techniques for a patient.

The data within the radiotherapy file is not limited to the external radiation therapy as other treatments may impact the radiation therapy, such as medical oncology treatment (chemotherapy), interventional oncology treatment (e.g. cryotherapy, microwave therapy, or embolic therapy), or other non-treatment procedures, such as labs and appointments with other medical professionals. The radiotherapy treatment file may include data specific to one or more patient's radiotherapy treatment. The radiotherapy treatment file may include any combination of one or more of: a patient identifier, patient's electronic health data records, medical images (e.g., CT scans, 4D CT scans, MRIs, and x-ray images), treatment-specific data (e.g., arc information or treatment type), target organ (e.g., specification and location data to identify the tumor to be eradicated), treatment plan, etc. Additional examples may include non-target organs, dosage-related calculations (e.g., radiation dose distribution within an anatomical region of the patient), and/or radiotherapy machine specific information (e.g., couch-gantry orientations, machine trajectory, control points, dose distributions, and/or arc information).

The analytics server **110** may use the patient identifier within the radiotherapy treatment file to identify a particular patient and retrieve additional information regarding said patient. For instance, the analytics server **110** may query the medical records database **120** to identify medical data associated with the patient. For instance, the analytics server may query data associated with the patient's anatomy, such as physical data (e.g., height, weight, and/or body mass index), and/or other health-related data (e.g., blood pressure or other data relevant to the patient receiving radiotherapy treatment). The analytics server **110** may also retrieve data associated with current and/or previous medical treatments received by the patient (e.g., prior treatment fractions, or data associated with the patient's previous surgeries).

The analytics server **110** may analyze the data received and generate additional queries accordingly. For instance, the analytics server **110** may retrieve data associated with one or more medical (or other) devices needed for the patient. The analytics server may retrieve data indicating that the patient suffers from a respiratory medical condition. As a result, the analytics server **110** may generate and transmit a query to the radiotherapy machine **141,** or the system administrator computer **150** to identify whether the patient uses/needs a ventilator.

If necessary, the analytics server **110** may also analyze the patient's medical data records to identify the needed patient attributes. For instance, the analytics server **110** may query a database to identify the patient's BMI. However, because many medical records are not digitalized, the analytics server **110** may not receive the patient's BMI value using simple query techniques. As a result, the analytics server **110** may retrieve the patient's electronic health data and may execute one or more analytical protocols (e.g., natural language processing) to identify the patient's body mass index. In another example, if the analytics server **110** does not receive tumor data (e.g., end-points), the analytics server **110** may execute various image recognition protocols and identify the tumor data.

Another example of a patient attribute may include specific tumor locations. More specifically, this data may indicate the primary tumor location with respect to the patient's centerline. This data may be inputted by the treating oncologist or may be analyzed using various image recognition or segmentation methods executed on the patient's medical images.

Another example of a patient attribute may include whether the patient uses prosthesis (e.g., hip or femoral head prosthesis). This attribute may result in a change of the patient's treatment (e.g., patients with these conditions might require a special treatment).

The analytics server **110** may use various application-programming interfaces (APIs) to communicate with different features described herein. As used herein, an API refers to a computing interface that uses connector programming code to act as a software intermediary between at least two computing components/features described herein. The API may automatically and/or periodically transfer various calls, instructions, and/or requests among different features of the system **100.** Using different APIs, the analytics server **110** may automatically transmit and/or receive calls and instructions.

Additionally, or alternatively, the analytics server **110** may use a content delivery network (CDN) to ensure data integrity when communicating with different features described in the system **100.** As described herein, a CDN refers to a distributed delivery network of proxy servers/nodes that uses multi-layered delivery methods/systems to transmit data (e.g., Akamai). The analytics server **110** may use a CDN when communicating various calls/instructions with the network **130** and/or the local database **111.**

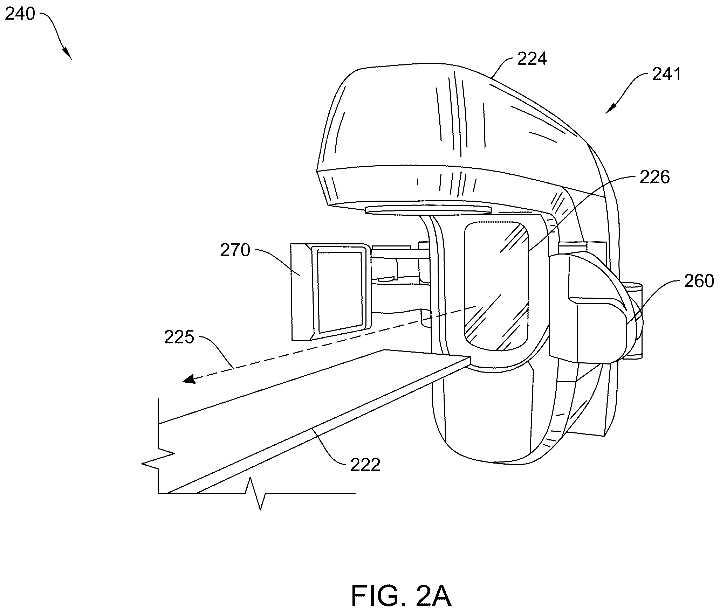

The radiotherapy machine **141** may also include a couch (shown as couch **222 in** **FIG. 2A****)** to align the patient in a designated position before the treatment begins. The designated position may be identified, calculated, and/or retrieved by the analytics server **110.** For example, the analytics server **110** may retrieve patient data from the medical records database **120,** analyze the data, and utilize the analyzed data to position the patient on the couch. In some embodiments, the doctor identifies how to align the patient on the couch to optimize the treatment therapy. The radiotherapy machine **141** directs radiation at specified locations of the patient resting on the couch during treatment. The specified locations may be selected by the technician operating the radiotherapy machine **141,** the pendant **142,** and/or the system administrator computer **150.** The analytics server **110** may also specify the locations on the patient to direct the radiation.

The radiotherapy machine **141** may also include an x-ray emitter and an x-ray receiver. The x-ray emitter may be disposed on a gantry of the radiotherapy machine **141** and may be configured to provide x-ray (e.g., a penetrating form of high-energy electromagnetic radiation) waves. The x-ray emitter emits x-ray waves to the patient who is positioned on the couch. The x-ray receiver may be disposed opposed to the x-ray emitter. The x-ray waves received by the x-ray receiver generate an imprint (e.g., image) of the patient's internal anatomy. Although the example embodiment recites the use of x-ray imaging, an alternative configuration for the radiotherapy machine may include an additional or other medical imaging apparatus (e.g., fluoroscopy apparatus, MRI, etc.).

The radiotherapy machine **141** may also include a set of cameras (e.g., camera on an end of the couch, gantry camera, ceiling camera, or other cameras placed within the radiotherapy room). The analytics server **110** may retrieve a live feed of the couch (e.g. with or without the patient) from the set of cameras and provide it to a display screen disposed on the radiotherapy machine **141.** The set of cameras may also directly communicate with the radiotherapy system **140.**

The radiotherapy machine **141** may also include a gantry that may be in communication with the analytics server **110.** If the radiotherapy machine **141** is in operation, the gantry may rotate relative to the radiotherapy machine **141** to begin the treatment of the patient. The analytics server **110** may use the live feed of the set of cameras to control and/or stop the movement of the gantry. During operation, the analytics server **110** may instruct the display screen of the radiotherapy machine **141** to display the live feed of the set of cameras and/or a designated page to the display screen of the radiotherapy machine **141.** In various embodiments, the analytics server **110** may not instruct the display screen of the radiotherapy machine **141** to activate the display screen of the radiotherapy machine **141** during operation. In some embodiments, the display screen of the radiotherapy machine **141** may be a touch screen and may control the pages generated by the analytics server **110** through the display screen of the radiotherapy machine **141.**

The radiotherapy machine **141** may have a default home position. Before the patient receives treatment, the radiotherapy machine **141** may be reset to a position that is ergonomically desirable for the patient. The default home position may also be optimized to receive a new patient or allow the patient to more easily get off the couch.

As discussed in greater detail herein, the radiotherapy machine **141** may also be controlled by the pendant **142.** The pendant **142** may be connected to the radiotherapy machine **141** through wired or wireless communications according to, for example, Bluetooth specification sets, or another standard or proprietary wireless communication protocol. In various embodiments, the pendant **142** may be connected to the radiotherapy machine **141** through a wired connection or be integrated into the radiotherapy machine **141.** The pendant **142** may also be in communication with the analytics server **110.** The pendant **142** may adjust the positioning of the radiotherapy machine **141** through a selection of buttons that axially actuates the radiotherapy machine **141** (e.g., couch) towards a desired direction. The technician may also use the pendant **142** to initialize the radiotherapy machine **141,** and/or to have the radiotherapy machine **141** return to its home position.

The pendant **142** may also allow the technician to input patient information, which may be then communicated to the radiotherapy machine **141,** the analytics server **110,** and/or the medical records database **120.** The pendant **142** may be used to control one or more of the steps of the setup and/or treatment of the patient.

The pendant **142** may also include a touch pad (e.g., tactile sensor). The touch pad may be used to control various pages generated by the analytics server **110.** For example, the user may use the touch pad of the pendant **142** to control a page, generated by the analytics server **110,** to proceed through the workflow steps of the radiotherapy setup and/or treatment. In some embodiments, the page may be controlled through a touch screen on the radiotherapy machine **141** or elsewhere in the treatment room.

As discussed in greater detail herein, the radiotherapy system **140** may further include accessories that assist in the radiotherapy setup and/or treatment of the patient. The analytics server **110** may communicate with the radiotherapy system **140** to select which accessories are required. The analytics server **110** may also communicate with the medical records database **120,** and/or the system administrator computer **150** to determine which accessories are required. For example, a patient may require a specific accessory for their radiotherapy setup and/or treatment. The analytics server **110** may retrieve that the patient requires a specific accessory by accessing the medical records database **120** and communicate that information to the radiotherapy system **140.** The accessories may be in communication with the radiotherapy system **140.** For instance, as will be described below, the analytics server may use RFID tags to scan and identify the location and/or presence of various accessories. In some embodiments, the accessories are wired or otherwise integrated into the radiotherapy machine **141.**

The local database **111** associated with the analytics server **110,** the medical records database **120,** and the radiotherapy machine **141** are capable of storing information in various formats and/or encrypted versions. The information may include data records associated with various patient information, user preferences, a set of prompts (e.g., question, query, and inquiry), attributes associated with various pages to be generated by the analytics server **110,** and the like. The medical records database **120,** may have a logical construct of data files, which are stored in non-transitory machine-readable storage media, such as a hard disk or memory, controlled by software modules of a database program (e.g., structured query language (SQL)), and a database management system that executes the code modules (e.g., SQL scripts) for various data queries and management functions.

The system administrator computer **150** may represent a computing device operated by a system administrator. The system administrator computer **150** may communicate with the analytics server **110.** The system administrator computer **150** may be configured to display various analytic metrics where the system administrator can monitor gantry movement, patient information, and modify various thresholds/rules described herein. The system administrator computer **150** may be configurable to display certain analytics metrics when specific thresholds/rules have been exceeded. For example, the system administrator computer **150** may be alerted if a patient has moved a specified amount during treatment. The analytics server **110** may allow the system administrator computer **150** to also control and/or override the settings of the radiotherapy machine **141,** and/or the pendant **142.** For instance, an administrator may revise the minimum distance threshold and/or customize any feature on the pages described herein (e.g., move features within the page, color, font, shape, size, or the order of display for one or more pages).

The analytics server **110** may configure the system administrator computer **150** to review any and/or all commands made through the radiotherapy system **140** at specified process steps. The system administrator computer **150** may then allow or reject the commands made through the radiotherapy system **140.** For example, before the technician starts the patient treatment using the radiotherapy machine **141,** the analytics server **110** may first prompt the system administrator computer **150** to review the radiotherapy machine **141** parameters for approval. Once approved, the technician may then control the radiotherapy machine **141.**

The analytics server **110** may configure the system administrator computer **150** to review any and/or all reading and/or (over)writing of patient data to and/or from the medical records database **120.** For example, before the technician may (over)write patient information collected by the radiotherapy machine **141,** the analytics server **110** may first prompt the system administrator computer **150** to review the patient information before it is stored in the medical records database **120.**

As discussed in greater detail herein, the analytics server **110** may generate various pages that are designed to interact with the individual operating the radiotherapy machine **141,** and/or the system administrator computer **150.** The analytics server **110** may then generate various interactive pages and may instruct the network **130** to incorporate the generated pages displayed on the radiotherapy machine **141.**

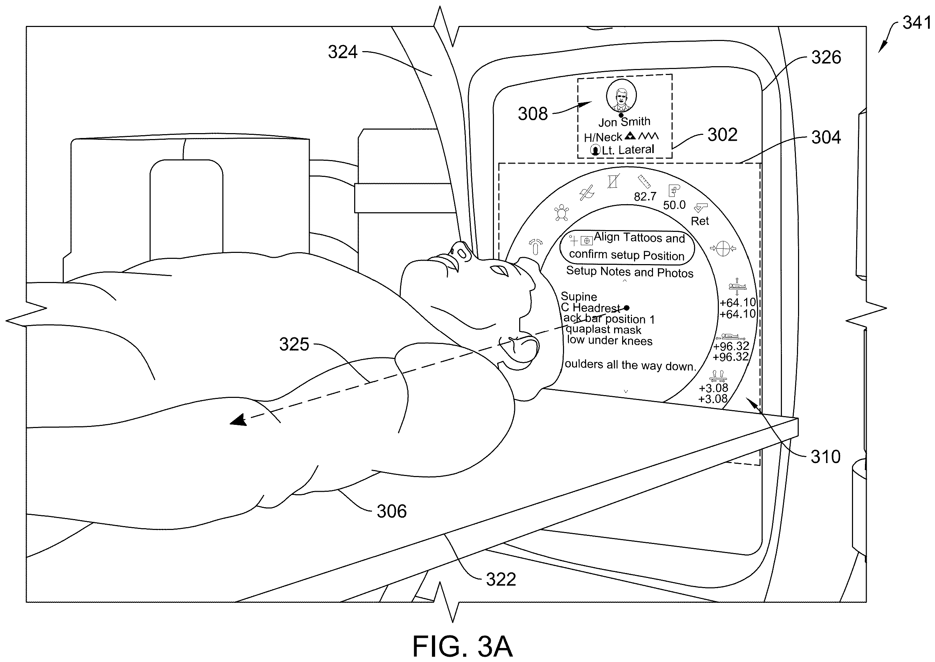

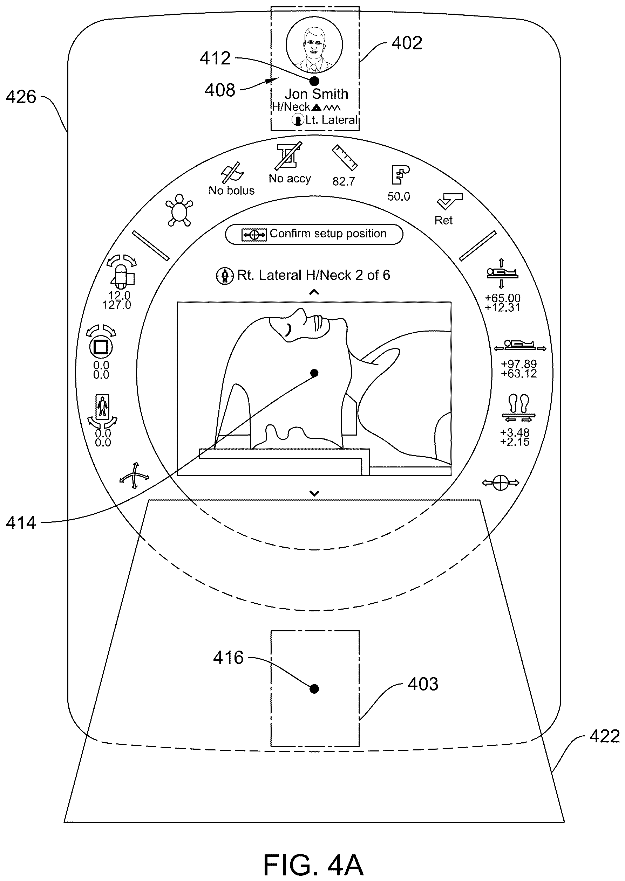

For example, in at least some embodiments of the systems and methods described herein, the analytics server **110** may present for display one or more sets of icons on a display screen coupled to the radiotherapy machine **141** to present radiotherapy information related to the radiotherapy treatment or radiotherapy setup (e.g., alignment information, patient information, etc.). While the analytics server **110** is described as presenting icons on the display screen generally, it should be understood that the systems and methods described herein extend to any graphical component, such as, but not limited to, an indicator, figure, avatar, badge, symbol, marker, shape, object, emblem, arrow, direction, text, or any combination thereof. During alignment, such as during rotation of the gantry about a central axis of the radiotherapy machine **141,** one or more icons within the set of icons may be hidden from a user's view (e.g., a technician, a patient, a medical practitioner) due to a location of the display screen relative to the couch or other object (as shown in **FIGS. 3A-3B** and **4A-4D).** Additionally, or alternatively, during rotation of the gantry-and by extension, the display screen-the one or more icons displayed on the display screen may be oriented, such that they are unreadable due to being statically displayed on the display screen (e.g., the icons may be presented in an upside-down orientation when the gantry is rotated 180° from a default, upright position). In such conditions, the analytics server may dynamically adjust a position and/or rotation of the one or more icons on the display screen, such that the one or more icons are maintained in a readable orientation and/or position regardless of an orientation and/or position of the gantry of the radiotherapy machine **141.** In embodiments in which the one or more icons may be obfuscated from view by a component of the radiotherapy system **140** (e.g., the couch), the analytics server **110** may dynamically adjust a position from one location on the display screen to a second position on the display screen, so as to maintain the one or more icons in a visible location during rotation of the gantry.

**FIG. 2A** illustrates, as described above, a radiotherapy system **240,** including a radiotherapy machine **241** that includes a gantry **224** that rotates in a direction **228** (which may be clockwise or counterclockwise) about a center axis **225** of the radiotherapy machine **241.** In some embodiments, the radiotherapy system **240** may be substantially similar to the radiotherapy system **140** of **FIG. 1****.** Likewise, the radiotherapy machine **241** may be substantially similar to the radiotherapy machine **141** of **FIG. 1****.** The radiotherapy machine **241** may further include a display screen **226** positioned on or within the gantry **224.** The display screen **226** may be configured to display for view to a user (e.g., a technician, patient, and/or a medical practitioner) relevant information related to the radiotherapy procedure and/or the radiotherapy setup procedure before, during, and/or after the radiotherapy procedure and/or the radiotherapy setup procedure.

The radiotherapy machine **241** may also include an imager **270** and an emitter **260** to facilitate visualizing (using X-ray imaging) the location of a patient prior to and during the administration of the radiotherapy to the patient. The radiotherapy system **240** may additionally include a couch **222,** upon which the patient may be placed before and during the administration of the radiotherapy. In some embodiments, the couch **222** may be translated relative to the radiotherapy machine **241** (e.g., along the center axis **225** and horizontally perpendicular to the center axis **225)** and/or rotated (e.g., yaw, pitch, roll). For example, the couch **222** may rotate relative to the radiotherapy machine **241** about the center axis **225** (e.g., roll). The couch **222** may rotate relative to the radiotherapy machine **241** about an axis extending horizontally perpendicular to the center axis **225** (e.g., pitch). In some embodiments, the couch **222** may rotate relative to the radiotherapy machine **241** about an axis extending vertically perpendicular to the center axis **225** (e.g., yaw). Likewise, the radiotherapy machine **241** may be rotated axially about the center axis **225,** and relative to the couch **222,** before, during, and/or after the radiotherapy setup and/or administration of the radiotherapy treatment.

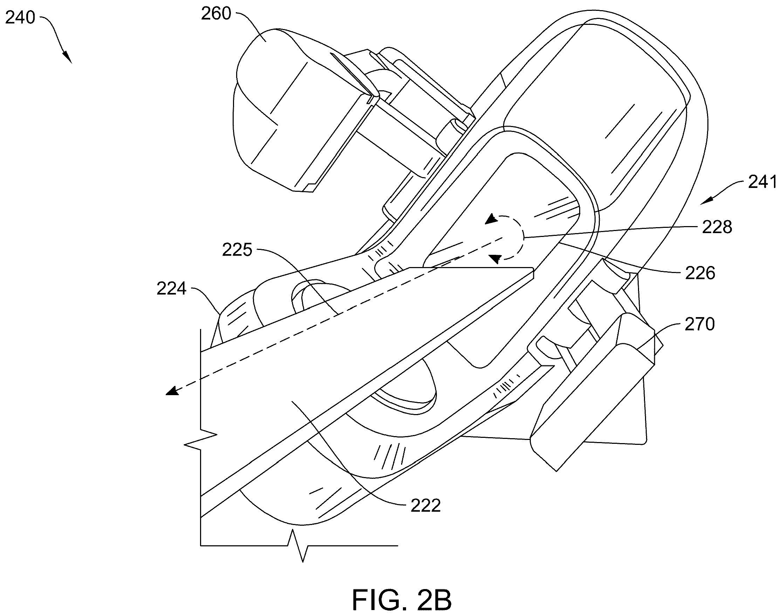

For example, as illustrated in **FIG. 2B****,** the radiotherapy machine **241** is shown in a rotated position about the center axis **225.** Relative to the default position of the radiotherapy machine **241** (as shown in **FIG. 2A****),** the radiotherapy machine **241 in** **FIG. 2B** is shown at a position that is rotated about 135.5° counterclockwise. Because the display screen **226** is coupled to the gantry **224** of the radiotherapy machine **241,** the display screen **226** is also rotated from a default, upright position (as shown in **FIG. 2A****)** at about 135.5° counterclockwise. Though the gantry **224** and the display screen **226** are shown rotating counterclockwise, it is understood that the gantry **224** and the display screen **226** may additionally or alternatively rotate clockwise from the default position shown in **FIG. 2A****.**

During a radiotherapy treatment procedure, as shown in **FIG. 3A****,** a patient **306** is placed upon the couch **322** (which may be substantially similar to the couch **222** of **FIG. 2A****),** which is positioned near the radiotherapy machine **341** (which may be substantially similar to the radiotherapy machine **241** of **FIG. 2A****).** Before and/or during the treatment, and/or during radiotherapy setup, a set of icons (e.g., graphical/textual icons) may be presented for display on the display screen **326** (which may be substantially similar to the display screen **226** of **FIG. 2A****).** In some embodiments, the icons displayed may be divided into two separate groups of subsets. The grouping may be performed using predefined criteria (e.g., in accordance with the content or location of the icon). In some embodiments, one group (e.g., one or more icons) may appear to rotate with the gantry **324** (which may be substantially similar to the gantry **224** of **FIG. 2A****)** and the display screen **326,** while another group of icons may appear to be stationary.

In a non-limiting example shown in **FIG. 3A****,** a subset of icons **310** of the set of icons may be presented for display in an area **304** on the display screen **326.** A second subset of icons (such as the second subset of icons **308** depicted in **FIG. 3A****)** may additionally or alternatively be presented for display at a first position **302** of the display screen **326.**

The second subset of icons **308** may include personally identifiable information related to the patient **306,** the radiotherapy setup and/or the radiotherapy treatment. For example, the second subset of icons **308** may include a visual graphic of the patient **306** such that a medical practitioner may visually confirm an identity of the patient **306** and verify that the radiotherapy treatment being administered is the correct radiotherapy treatment. Additionally, or alternatively, the second subset of icons **308** may include text related to the patient **306** and/or personally identifiable information of the patient **306.** Personally identifiable information associated with the patient **306** that may be displayed in the second subset of icons **308** on the display screen **326** may include, but is by no means limited to, a name, age, height, weight, type of treatment to be administered, emergency contact information, insurance information, a Social Security number, driver's license numbers, passport details, addresses, email addresses, phone numbers, credit card or bank account information, biometric data (such as fingerprints or facial recognition data), medical records, dates of birth, employment records, and/or educational records.

The subset of icons **310** may include information related to the setup of the radiotherapy machine **341,** the couch **322,** previous treatments, and/or the radiotherapy treatment more generally. For example, the subset of icons **310** may include visual and/or text graphics related to a current position of the radiotherapy machine **341** and/or the couch **322,** and a desired position of the radiotherapy machine **341** and/or the couch **322.** In some embodiments, the subset of icons **310** may include visual and/or text/numerical indicators that indicate a positional change to be applied to one or more of the couch **322** and/or the radiotherapy machine **341,** such that the couch **322** is appropriately aligned relative to the radiotherapy machine **341** during setup, for administration of the radiotherapy treatment. In some embodiments, one or more icons within the subset of icons **310** may be dynamically updated as the radiotherapy machine **341** and/or the couch **322** are positionally adjusted.

As shown in **FIG. 3A****,** the second subset of icons **308** of a set of icons is displayed in the first position **302** of the display screen **326.** In one embodiment shown in **FIG. 3A****,** the first position **302** is positioned at the top of the display screen **326** when the gantry **324** is in the default position. The absolute location of the first position **302** relative to the display screen **326** may be maintained during rotation of the gantry **324,** as shown in **FIGS. 3A-3D****.** In some embodiments, the second subset of icons **308** are maintained within the first position **302** of the display screen **326,** regardless of the position of the gantry **324** during rotation movement. Without a dynamic adjustment of the second subset of icons **308** within the first position **302** of the display screen **326,** the second subset of icons **308** of the set of icons would maintain its orientation relative to the display screen **326** and be illegible when rotated. As such, in the methods and systems described herein, an analytics server (e.g., the analytics server **110 of** **FIG. 1****)** may provide instructions to the display screen **326** to dynamically relocate and/or dynamically reorient the second subset of icons **308** of the set of icons during rotation of the gantry **324,** such that an orientation (e.g., the default, upright orientation) of the second subset of icons **308** of the set of icons relative to a reference object/position/location (e.g., the couch **322,** gravity, etc.) is maintained during rotation, thus appearing to rotate with the gantry **324.** In this way, the second subset of icons **308** is maintained in a legible orientation regardless of the position of the gantry **324.** The analytics server may also provide instructions to the display screen **326** to dynamically adjust, relocate, and/or dynamically reorient the subset of icons **310** of the set of icons during rotation of the gantry **324,** such that an orientation (e.g., the default, upright orientation) of the subset of icons **310** of the set of icons relative to a reference object/position/location (e.g., the couch **322,** the display screen **326,** gravity, etc.) is maintained during rotation, in this instance, appearing to be stationary relative to the display screen **326.** In this way, the subset of icons **310** is maintained in a legible orientation regardless of the position of the gantry **324,** thus providing a user of the gantry **324** with a user-friendly and intuitive display during setup of the gantry **324** in preparation of administration of the radiotherapy. This user-friendly and intuitive display decreases setup time and effort, resulting in faster radiotherapy treatment administration and/or faster radiotherapy setup.

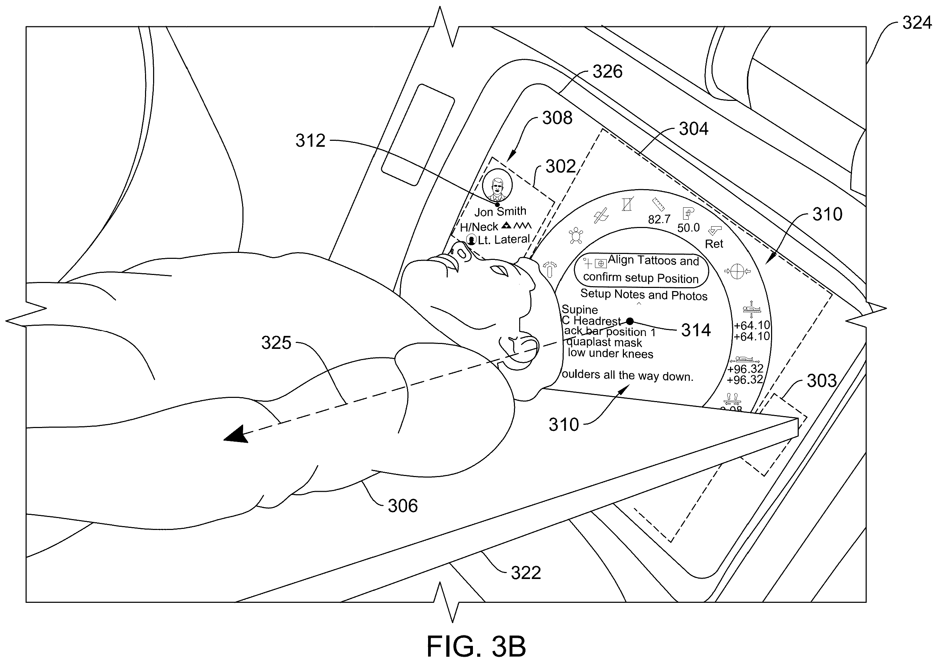

Turning now to **FIGS. 3B-3D****,** the gantry **324** is shown rotating counterclockwise to various orientations relative to a reference, such as a reference orientation (e.g., the upright orientation shown **FIG. 3A****),** a reference position (e.g., the upright position as shown in **FIG. 3A****,** vertical), a reference object (e.g., the couch **322,** a support such as the ground, a level, gravity), etc. In each rotated orientation of the gantry **324** in **FIGS. 3B-3D****,** the second subset of icons **308** are maintained in an orientation and/or position relative to the reference to maintain legibility. In **FIG. 3B****,** the gantry **324** is rotated to a position at about 45° counterclockwise from a first, upright position of the gantry **324** (e.g., as shown in **FIG. 3A****).** The analytics server, which is communicably coupled to the radiotherapy machine **341,** may receive an indication of a movement of the gantry **324,** such as during movement of the gantry **324** from its position in **FIG. 3A** to its position in **FIG. 3B****.** The indication of the movement of the gantry **324** may be an indication that the gantry **324** is currently rotating (or will rotate) from the upright position to the second position. The indication of movement may include one or more movement parameters that define the movement of the gantry **324.** Such movement parameters may include, but are not limited to, any combination of one or more of: a speed of rotation of the movement of the gantry, a direction of rotation of the movement of the gantry, an acceleration of the movement of the gantry, and/or an angle of rotation of the movement of the gantry.

The speed of rotation of the gantry may refer to how fast the gantry **324** is spinning or rotating around a central axis (e.g., the center axis **325).** The speed of rotation may be measured in terms of angular velocity of the gantry **324,** quantifying the angle turned per unit of time, such as radians per second, degrees per second, or revolutions per minute (RPM). The speed of rotation of the gantry **324** may depend on its mass distribution, the forces acting on it, and the energy imparted by the radiotherapy machine **341** to initiate or sustain the rotation. Such parameters may be used by the analytics server in determining the speed of rotating of the gantry.

The direction of rotation of the gantry **324** may refer to a specific path or orientation in which the gantry **324** is rotating around the central axis (e.g., the center axis **325).** The direction of rotation can be clockwise or counterclockwise when viewed from a particular reference point (such as from the couch **322).** This parameter may depend on the mechanical configuration of the gantry system, the applied forces, and the intended operation of the gantry **324.** Such parameters may be analyzed by the analytics server to determine the direction of rotation of the gantry **324.**

The acceleration of the movement of the gantry **324** may refer to the rate at which the speed or velocity of the gantry's rotation or linear movement changes over time. Acceleration can be expressed in units such as meters per second squared (m/s²) or radians per second squared for angular motion. The acceleration of the movement may depend on the torque applied, the resistance encountered, and/or the inertia of the gantry **324.** The analytics server may use any combination of one or more of these parameters to monitor changes in the motion dynamics of the radiotherapy machine **341** and determine the acceleration of the movement of the gantry **324.**

The angle of rotation of the movement of the gantry **324** may refer to the total angular displacement the gantry **324** achieves as it rotates around a central axis (e.g., the center axis **325).** This angle may be measured in degrees, radians, or revolutions, and can indicate how far the gantry has rotated from its initial position. In some embodiments, the upright position of the gantry **324,** as shown in **FIG. 3A****,** may be considered the initial position and measured at 0°, 0 rad, etc. The angle of rotation may depend on the direction of rotation, duration of rotation, the acceleration of the rotation, the applied torque, and/or the stopping forces acting on the gantry **324.** Such parameters may be utilized by the analytics server to determine the rotational progress of the gantry **324** during movement and, thereby, determine a total acceleration or deceleration of the gantry **324.**

The analytics server may use one or more of the movement parameters above to calculate one or more corresponding icon movement parameters to use in dynamically adjusting, moving, and/or relocating (e.g., rotating) the second subset of icons **308** relative to the display screen **326.** In other words, as the display screen **326** rotates with the gantry **324** in accordance with the one or more movement parameters, the analytics server may determine corresponding icon movement parameters to apply to the second subset of icons **308,** such that the second subset of icons **308** appear to maintain a first orientation (e.g., upright), as shown in **FIGS. 3A-3D****.**

In some embodiments, the icon movement parameters are opposite the movement parameters of the gantry **324.** For example, as the gantry **324** rotates counter-clockwise at an angle of rotation (e.g., approximately 45° counter-clockwise, as shown by the movement of the gantry **324** between **FIGS. 3A-3B****),** the second subset of icons **308** may be relocated/adjusted in a clockwise rotation within the first position **302** about a secondary datum point **312** at an opposite angle of rotation (e.g., approximately 45° clockwise) to "cancel out" the opposite rotation of the gantry **324** and thereby maintain the upright orientation while the secondary datum point **312** (and by extension, the second subset of icons 308) rotates about a center datum point **314**/center axis **325.** The second subset of icons **308** may rotate about the secondary datum point **312,** which may be associated with (e.g., defining or defined by) the first position **302.** For example, the secondary datum point **312** may be in a center of the first position **302.** In some embodiments, the secondary datum point **312** is a center point of the second subset of icons **308** and/or the first position **302.** In some embodiments, the gantry **324** (and by extension, the display screen **326)** rotates about the center axis **325,** which may extend from the center datum point **314.** As the gantry **324** (and by extension, the display screen **326)** rotates about the center axis **325,** the secondary datum point **312** may rotate about the center datum point **314.** In this way, as the second subset of icons **308** rotates about the secondary datum point **312** consistent with the determined icon movement parameters, the second subset of icons **308** are relocated/adjusted/rotated at an angle corresponding to the angle of rotation of the gantry **324** but in the opposite direction, such that they appear to be rotating consistent with the movement of the gantry **324** while maintaining an orientation, such as an upright orientation. While the second subset of icons **308** is shown rotating clockwise to counteract the counterclockwise rotation of the gantry **324,** it is understood that in embodiments in which the rotation of the gantry **324** is clockwise, the second subset of icons **308** may rotate counterclockwise to counteract the clockwise rotation of the gantry **324.** In like manner, the second subset of icons **308** appear to rotate consistent with the movement of the gantry **324.** As described herein, in either implementation, the second subset of icons **308** rotates in the direction opposite the gantry **324,** thus appearing to rotate consistent with the movement of the gantry **324.**

In some embodiments, the gantry **324** is able to laterally adjust position. In such embodiments, the movement of the gantry **324** is not confined to rotation. In such embodiments, the analytics server may determine one or more additional movement parameters, such as any combination of one or more of: lateral direction, speed, position, etc. The analytics server may determine icon movement parameters corresponding to the lateral movement to apply to the second subset of icons **308** to adjust a position of the second subset of icons **308** during movement of the gantry **324,** such that the second subset of icons **308** appear to not be moving laterally with the movement of the gantry **324.** Upon determining the icon movement parameters, the analytics server may transmit corresponding control signals to the display screen to adjust a display of the second subset of icons **308** in accordance with the determined icon movement parameters. In general, the position and/or orientation of the second subset of icons **308** may be adjusted such that the second subset of icons **308** appear to not be moving and/or to not be rotating with the movement (e.g. any kind of movement) of the gantry **324.**

Additionally, or alternatively to the second subset of icons **308,** the display screen **326** may have displayed thereon the subset of icons **310.** The subset of icons **310** may be positioned circumferentially about the center datum point **314,** such as shown in **FIGS. 3A-3D****.** The analytics server may determine additional or alternative icon movement parameters corresponding to the movement parameters of the gantry **324** to apply to the subset of icons **310,** such that the subset of icons **310** appears to not be rotating relative to the movement of the gantry **324.** For example, without instructions to relocate the subset of icons **310,** during rotation of the gantry **324** (and by extension the display screen **326)** the subset of icons **310** would become illegible because they would no longer be in the upright orientation. Accordingly, in some embodiments, the analytics server determines one or more icon movement parameters to apply to the subset of icons **310** to maintain an upright orientation of the subset of icons **310** regardless of the position and/or orientation of the display screen **326.** As shown by the subset of icons **310** between **FIGS. 3A-****3D,** the subset of icons **310** maintains a consistent orientation and position relative to a reference (e.g., the couch **322,** gravity, etc.), while the display screen **326** is rotated about the center datum point **314.** In this way, the subset of icons **310** appears not to be moving relative to the couch **322.** While the subset of icons **310** is shown rotating clockwise to counteract the counterclockwise rotation of the gantry **324,** it is understood that in embodiments in which the rotation of the gantry **324** is clockwise, the subset of icons **310** may rotate counterclockwise to counteract the clockwise rotation of the gantry **324.** In such manner, the subset of icons **310** appears to be stationary with respect to the gantry **324.** As described herein, in either implementation, the subset of icons **310** rotates in the direction opposite the gantry **324,** thus appearing to be stationary relative to the gantry **324.** In general, the position and/or orientation of the subset of icons **310** may be adjusted such that the subset of icons **310** appear to not be moving and/or to not be rotating with the movement (e.g. any kind of movement) of the gantry **324.**

In some embodiments, the analytics server periodically (e.g., 1 ms, 60 hertz, etc.) relocates the second subset of icons **308** and/or the subset of icons **310** to continually update the orientation and/or position of the second subset of icons **308** and/or the subset of icons **310** consistent with the movement of the gantry **324.**

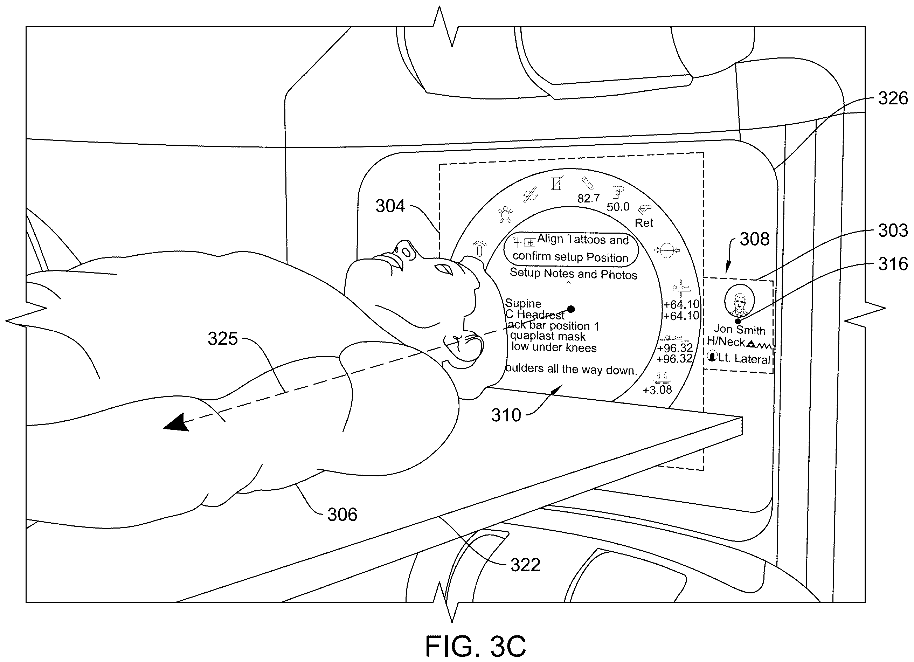

In some embodiments, during rotation of the gantry **324,** the second subset of icons 308 and/or the subset of icons **310** are maintained within bounded, respective areas. For example, the second subset of icons **308** may be positioned and maintained within the first position **302** during relocating of the second subset of icons **308.** Additionally, or alternatively, the subset of icons **310** may be positioned and maintained within the area **304** during relocation of the subset of icons **310.** Due to being maintained within the first position **302,** the second subset of icons **308** may, in some embodiments, be positioned in an area not visible to a user of the gantry **324.** For example, upon a continuation of rotation from **FIG. 3B****,** the second subset of icons **308** (while being maintained in the first position **302)** may be located behind the head of the patient **306** and then below the couch **322,** when viewed from the vantage point illustrated in **FIG. 3B****.** It may be desired to have the information displayed in the second subset of icons **308** visible and legible at all positions of the gantry **324.** As such, in some embodiments, the analytics server may remove the second subset of icons **308** from the first position **302** upon receiving an indication that the gantry **324** has rotated or moved beyond a first defined position. In at least one embodiment, the first defined position may be correlated to a rotational position (e.g., a number of angles) of the gantry **324** relative to the upright position (as shown in **FIG. 3A****).** In at least one embodiment, the gantry **324** may be in the first defined position in **FIG. 3B****.** As the gantry **324** continues a counterclockwise rotational movement (as shown in **FIG. 3C****),** the analytics server may transmit instructions to the display screen **326** to remove second subset of icons **308** from the first position **302** and display them at a second position **303** not obfuscated from view. In this way, the information presented by the second subset of icons **308** is not hidden from view beneath the couch **322** during rotation of the gantry **324** and remains visible to a user. By maintaining visibility of the second subset of icons **308** during setup of the gantry **324** in preparation for administration of the radiotherapy, the user can quickly and easily view all relevant data of the treatment regardless of the position of the gantry **324,** thus reducing setup time and the overall time of administration of the radiotherapy, resulting in quicker radiotherapies.

The indication that the gantry **324** has rotated beyond the first defined position may be generated and transmitted by various methods. For example, the gantry **324** may include one or more actuators for rotating the gantry **324.** The actuators may include feedback circuits to provide an indication of the position of the gantry **324.** In such embodiments, the feedback loop may provide a signal to the analytics server once the position has reached or passed the first defined position. The first defined position may be set by a user through a user interface, such as on a pendant (e.g., the pendant **142 of** **FIG. 1****)** and/or another electronic device (e.g., the system administrator computer **150 of** **FIG. 1****).** In some embodiments, the gantry **324** may interact with one or more sensors, such as a limit switch that provides an electronic signal to the analytics server once a limit (e.g., the first defined position) is reached.

**FIG. 3C** illustrates the second subset of icons **308** being displayed at/in the second position **303** upon the gantry **324** reaching the first defined position. As the gantry **324** continues to rotate counter-clockwise, as shown in the progression of the gantry **324 in** **FIGS. 3C-3D****,** the second subset of icons **308** continues to rotate in the second position **303** about a tertiary datum point **316** within the second position **303** such that the second subset of icons **308** maintains a static orientation (e.g., upright) as the tertiary datum point **316** orbits the center datum point **314** during rotation of the gantry **324** (and by extension the display screen **326),** thereby maintaining legibility.

In some embodiments, as the second subset of icons **308** is removed from the first position **302** and redisplayed at the second position **303,** the subset of icons **310** are maintained in a static orientation as well, by rotating each of the icons of the subset of icons **310** about the center datum point **314** as the gantry **324** rotates about the center axis **325.** The analytics server may transmit instructions to adjust a position of each pixel of each icon of the second subset of icons **308** and/or the subset of icons **310** to maintain the static orientation during rotation of the display screen **326.** The analytics server may take into account one or more positional parameters such as a distance from the center datum point **314,** a distance from the secondary datum point **312,** a distance from the tertiary datum point **316,** a speed of rotation of the gantry **324,** an acceleration of the gantry **324,** etc. In general, instructions may be transmitted to adjust a position of each pixel of any combination of the icons to maintain the static orientation and/or position of the icons during movement (e.g. any kind of movement ) of the gantry **324.**

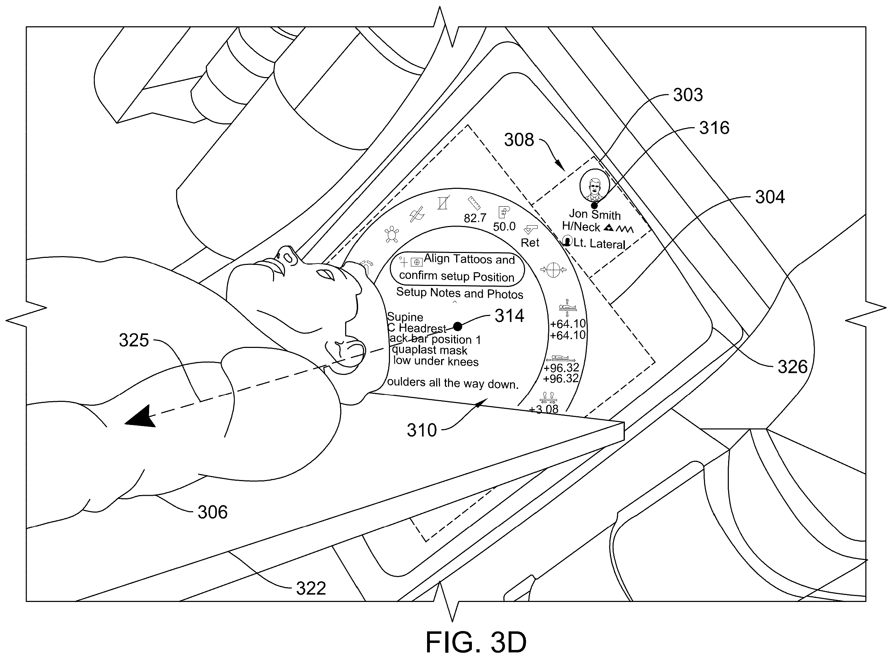

**FIG. 3D** illustrates the gantry **324** further rotating about the center axis **325** and the second subset of icons **308** and the subset of icons **310** further being adjusted in position/orientation, such that they maintain a static orientation, the second subset of icons **308** appearing to rotate consistent with the movement of the gantry **324** and/or the subset of icons **310** appearing to not be rotating relative to the movement of the gantry **324.**

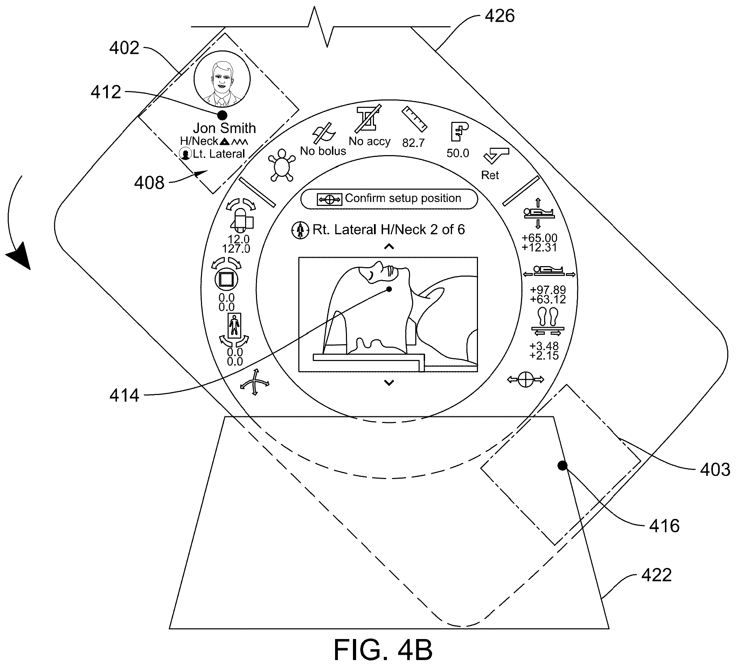

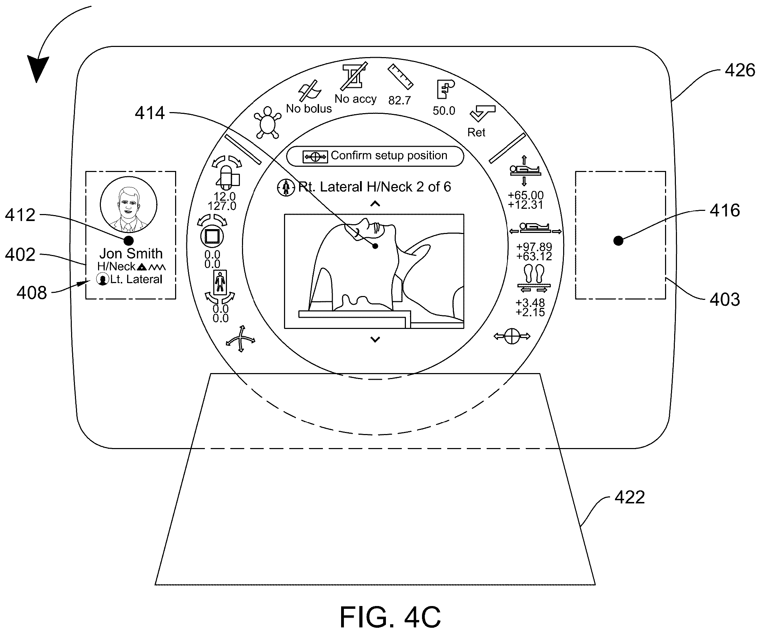

Turning now to **FIGS. 4A-4D****,** a front view of a display screen **426** (which may be substantially similar to the display screen **226** of **FIG. 2A****)** coupled (e.g., mounted, attached, fastened, adhered) to a gantry (e.g., the gantry **224** of **FIGS. 2A-3D****).** As shown in **FIG. 4A****,** a first position **402** (which may be substantially similar to the first position **302** of **FIG. 3A****)** of the display screen **426** is shown visible, and a second position **403** (which may be substantially similar to the second position **303** of **FIG. 3C****)** is hidden from view (shown as dashed lines) beneath/behind a couch **422** (which may be substantially similar to the couch **222** of **FIG. 2A****)** when the display screen **426** is in a first orientation (e.g., upright). As the display screen rotates counterclockwise about a center datum point **414** (which may be substantially similar to the center datum point **314** of **FIGS. 3B** and **3D****)** to a second orientation (as shown in **FIG. 4B****),** the first position **402** moves with a secondary datum point **412** (which may be substantially similar to the secondary datum point **312** of **FIG. 3B****)** and remains visible, while the second position **403** moves with a tertiary datum point **416** (which may be substantially similar to the tertiary datum point **316** of **FIGS. 3C-3D****)** and emerges from behind/beneath the occlusion of the couch **422.** In continuing the rotation (as shown in **FIG. 4C****)** the display screen **426** may reach a first defined position at which point, in response to reaching the first defined position, the analytics server may obfuscate and/or remove a second subset of icons **408** (which may be substantially similar to the second subset of icons **308** of **FIG. 3A****)** from being presented at the first position **402** on the display. The radiotherapy machine may include, in some embodiments, at least one position sensor, rotary encoder, and/or software-based positional tracking to detect when the gantry reaches the first defined position or a corresponding predefined angle. Upon reaching the first defined position, the analytics server may execute a GUI update (e.g., refresh, reload, add, and/or replace some or all of the GUI) by removing, dimming, greying out, or otherwise obfuscating certain icons (e.g., the second subset of icons **408).** This process can be implemented through software-driven GUI rendering techniques, such as adjusting transparency levels, altering contrast, or replacing specific icons with placeholders.

In some implementations, the system may allow configurable obfuscation rules, enabling technicians, users, and/or administrators to define which icons should be obfuscated, hidden, dimmed, and/or replaced at specific gantry positions. Additionally, obfuscation may be time-dependent, where the second subset of icons **408** gradually fade out from the first position 402 after a brief delay.

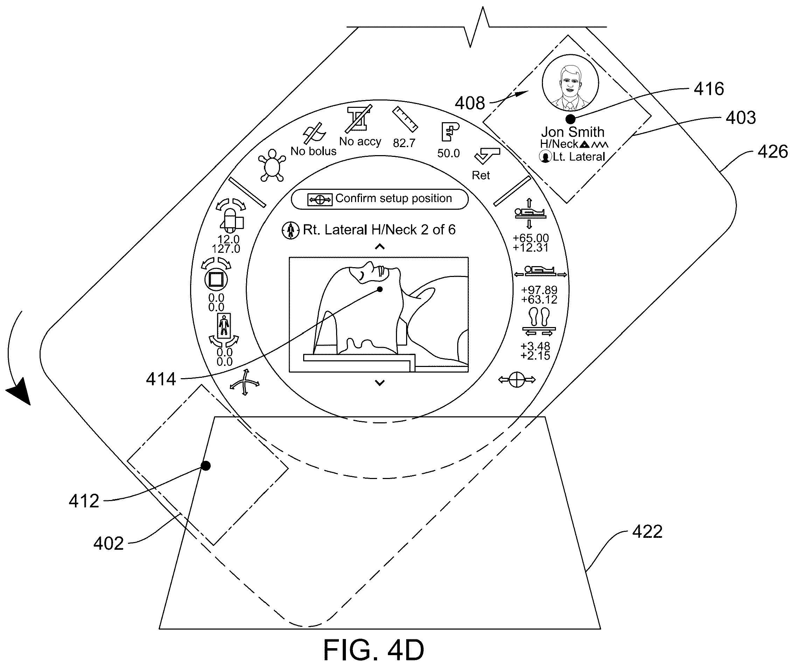

Upon removing or otherwise obfuscating the second subset of icons **408** from display at the first position **402,** the analytics server may present for display the second subset of icons **408** at the second position **403.** In some embodiments, the analytics server may transmit one or more control signals to the display screen **426** to present for display the second subset of icons **408** at the second position **403.** **FIG. 4D** illustrates the display screen **426** further rotated counterclockwise farther past the first defined position shown in **FIG. 4C****.** In some embodiments, the analytics server may transmit instructions to disable the display screen **426** upon the gantry reaching a defined location (e.g., a radiotherapy position used in administering the radiotherapy). Disabling the display screen may include removing power to the display screen **426.**

As described above, the radiotherapy machine may include position sensors, encoders, and/or feedback circuits integrated into the gantry's movement mechanism, allowing the system's processor to detect when the gantry reaches the defined location-such as a treatment position. Upon detecting that the gantry has reached the defined location, the analytics server transmits a control signal to disable the display screen **426,** either by powering it down, dimming the screen, or entering a low-power standby mode. The system may also store configurable parameters, allowing users to set and/or adjust the defined position for screen disabling based on treatment protocols, system setup, user preferences, and/or safety requirements.

In addition or alternative to fully powering down (e.g., disabling) the display screen when the gantry reaches the defined location, the system may implement several alternative methods. For example, an alternative approach involves dimming the screen to a lower brightness level or activating a dark mode. Another alternative may include entering a standby mode, in which the screen remains active but ceases to display specific elements, instead showing, for example, a minimal indicator confirming that the system is operational.

In some embodiments, the system initiates a context-based reconfiguration upon the gantry reaching the defined location, where the display automatically shifts to a different interface mode when the gantry reaches the defined position. Instead of disabling the display screen **426,** the GUI could transition to a simple status display with essential metrics such as treatment progress, patient positioning confirmation, and/or real-time safety alerts.

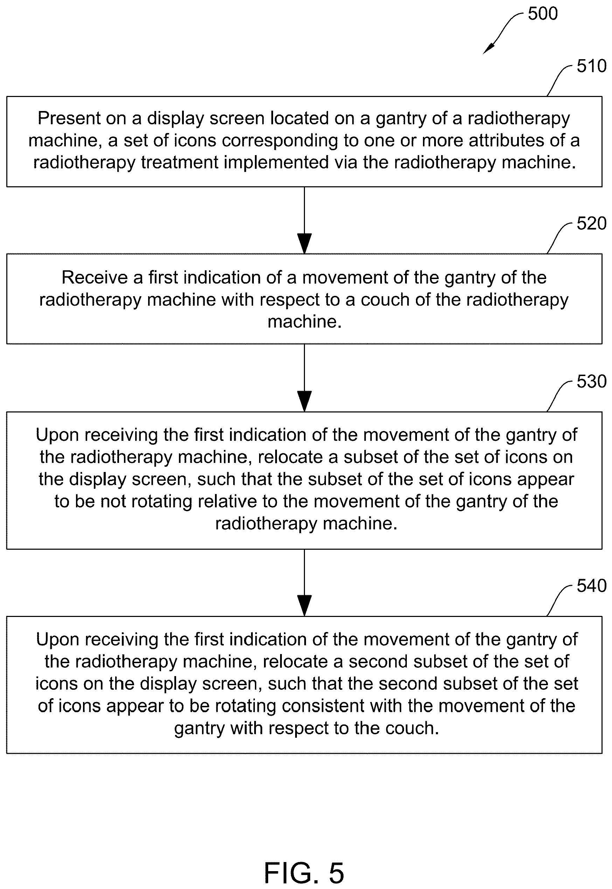

**FIG. 5** is a flowchart of an example method **500** for dynamically adjusting orientation and position of one or more icons on a display screen coupled to a radiotherapy machine. The method **500** may be executed by one or more processors of the one or more electronic described herein, such as the analytics server **110,** the pendant **142,** the system administrator computer **150,** the radiotherapy system **140,** the radiotherapy machine **141,** etc. The method **500** may be performed by the system in general. The one or more processors may execute instructions stored on computer-readable, non-transitory medium, which may cause the one or more processors to execute steps comprising the steps **510, 520, 530,** and/or **540.** While steps **510, 520, 530,** and **540** are shown in a specific order, it should be understood that the method **500** may comprise more, less, and/or different steps than those depicted in **FIG. 5****.** Likewise, the order of the steps **510, 520, 530,** and **540** are shown in **FIG. 5** for illustrative purposes. However, it is understood that the steps of method **500** may be performed in any number of configurations or orders without departing from the scope of the systems and methods described herein. By way of example, the method **500** may include only steps **510, 520,** and **530.** In some embodiments, the method **500** may include only steps **510, 520,** and **540.** In some embodiments, the method **500** may include steps **510, 520, 530,** and **540.**

At step **510,** one or more processors may present on a display screen located on a gantry of a radiotherapy machine, a set of icons corresponding to one or more attributes of a radiotherapy treatment implemented via the radiotherapy machine, and/or one or more attributes of a radiotherapy setup. As described herein, the one or more processors may transmit instructions to a display to display a set of icons comprised of one or more subset of icons, such as the first and subset of icons of **FIGS. 3A-4D****.** The icons may display information related to administration of a radiotherapy treatment to a patient, and/or related to radiotherapy setup. This data may include personal information of the patient and/or positioning data for a radiotherapy machine and/or a couch (e.g., the couch **222 of** **FIG. 2A****)** upon which the user is placed during setup and/or administration of the radiotherapy treatment.

At step **520,** one or more processors may receive a first indication of a movement of the gantry of the radiotherapy machine with respect to a couch of the radiotherapy machine. The gantry of the radiotherapy machine to which the display screen is coupled may be configured to move along or about one or more axes. By way of example, the gantry may be configured to rotate about a center axis (e.g., the center axis **225** of **FIG. 2A****)** to position the radiotherapy machine for administration of the radiotherapy and/or as part of setup.

When the gantry is moved, or an instruction is provided to the gantry which instructs the gantry to move, the one or more processors may receive an indication of the movement and/or instructions. This indication may come from a controller which sends control signals to the gantry to move. In some embodiments, the one or more processors receive the indication of movement of the gantry from feedback loop of one or more actuators coupled to the gantry and configured to adjust the position of the gantry. These feedback loops may provide data packets to the one or more processors with movement parameters of the gantry, such as any combination of one or more of: speed of rotation, angular acceleration, time of movement, etc.

At step **530,** upon receiving the first indication of the movement of the gantry of the radiotherapy machine, one or more processors may relocate and/or reorientate a subset of the set of icons on the display screen, such that the subset of the set of icons appear to be not rotating relative to the movement of the gantry of the radiotherapy machine. Upon receiving the indication of the movement of the gantry and the associated movement parameters of the gantry, the one or more processors may determine corresponding icon movement parameters to apply to the subset of the set of icons. In applying the determined corresponding icon movement parameters, the subset of icons are relocated and/or reorientated on the display screen such that they maintain an orientation relative to a reference (e.g., the couch, gravity, the ground, etc.), despite the movement and/or rotation of the display screen, which is coupled to the gantry. The icon movement parameters may be used to counter the movement of the gantry, such that the subset of icons appear to be static with respect to a reference (e.g., the couch, gravity, the ground, etc.), even though the gantry (and by extension, the display screen) are rotating about an axis.

The one or more processors may determine control signals in accordance with the icon movement parameters and transmit the control signals to the display screen, such that the display screen displays the subset of icons in accordance with the icon movement parameters.

At step **540,** upon receiving the first indication of the movement of the gantry of the radiotherapy machine, one or more processors may relocate and/or reorientate a second subset of the set of icons on the display screen, such that the second subset of the set of icons appear to be rotating consistent with the movement of the gantry. Upon receiving the indication of the movement of the gantry and the associated movement parameters of the gantry, the one or more processors may determine corresponding icon movement parameters to apply to the second subset of the set of icons. In applying the determined corresponding icon movement parameters, the second subset of icons is relocated on the display screen, such that they maintain an orientation relative to a reference (e.g., the couch, gravity, the ground, etc.), despite the movement and/or rotation of the display screen, which is coupled to the gantry. The icon movement parameters may be used to counter the movement of the gantry, such that the second subset of icons appear to be rotating consistent with the movement of the gantry with respect to a reference (e.g., the couch, gravity, the ground, etc.).

The one or more processors may determine control signals in accordance with the icon movement parameters and transmit the control signals to the display screen, such that the display screen displays the second subset of icons in accordance with the icon movement parameters.

### Example

A display screen on a gantry of a radiotherapy machine may present a GUI having a set of icons to assist an operator with guidance for setup and/or adjustments. During rotation of the gantry, the set of icons may be dynamically adjusted on a display screen. During the dynamic adjustment, the set of icons may be relocated and/or reorientated on the display screen such that they maintain an orientation relative to a reference (e.g., a couch within the radiotherapy system) despite the rotation of the display screen. In some implementations, the dynamic adjustment may include removing the set of icons from a first position and displaying them at a second position during rotation of the gantry to avoid obfuscating the set of icons behind a component of the radiotherapy system. As a result, the operator may continue to interact with the GUI in an efficient manner even though the display screen may be rotated from its original and upright position.

### Example clauses

Further aspects of these teachings are provided by the subject matter of the following clauses.

Clause 1. A computer-readable medium with instructions stored therein that when executed by one or more processors, cause the one or more processors to: present on a display screen located on a gantry of a radiotherapy machine, a set of icons corresponding to one or more attributes of a radiotherapy treatment implemented via the radiotherapy machine; receive a first indication of a movement and/or rotation of the gantry of the radiotherapy machine; and upon receiving the first indication of the movement and/or rotation of the gantry of the radiotherapy machine, relocate and/or reorientate a subset of the set of icons on the display screen, such that the subset of the set of icons appears to be not moving and/or rotating relative to a movement and/or rotation of the gantry of the radiotherapy machine.

Clause 2. The computer-readable medium of clause 1, wherein the instructions further cause the one or more processors to, upon receiving the first indication of the movement and/or rotation of the gantry of the radiotherapy machine, relocate a second subset of the set of icons on the display screen, such that the second subset of the set of icons appear to be rotating consistent with the movement of the gantry.

Clause 3. The computer-readable medium of clauses 1-2, wherein the instructions further cause the one or more processors to: remove the second subset of the set of icons from a first position when the gantry has rotated beyond a first defined position; and display at a second position the second subset of the set of icons previously removed when the gantry has rotated beyond a second defined position.

Clause 4. The computer-readable medium of clauses 1-3, wherein the second subset of the set of icons displays an identifier of a patient being treated.

Clause 5. The computer-readable medium of clauses 1-4, wherein the instructions further cause the one or more processors to: disable the display screen when the gantry is at a defined position.

Clause 6. The computer-readable medium of clauses 1-5, wherein the instructions further cause the one or more processors to: obfuscate at least one icon within the set of icons when the gantry reaches a defined position.

Clause 7. The computer-readable medium of clauses 2-6, wherein relocating of the second subset of the set of icons is consistent with a movement parameter of the movement of the gantry of the radiotherapy machine.

Clause 8. The computer-readable medium of clauses 1-7, wherein the instructions further cause the one or more processors to: receive a second indication of the movement parameter of the gantry of the radiotherapy machine; determine an icon movement parameter for the second subset of the set of icons, wherein the icon movement parameter corresponds to the movement parameter of the gantry; and upon receiving the first indication of the movement and/or rotation of the gantry of the radiotherapy machine, relocate and/or reorientate the second subset of the set of icons on the display screen in accordance with the icon movement parameter, such that the second subset of the set of icons appear to be rotating with the movement of the gantry, e.g. while maintaining a first orientation.

Clause 8.1. The computer-readable medium of clauses 1-8, wherein the instructions further cause the one or more processors to adjust a first orientation of the second subset of the set of icons with respect to the display screen at a first angle of rotation opposite of a second angle of rotation of the movement of the gantry such that a second orientation of the second subset of the set of icons is maintained when relocating the second subset of the set of icons on the display screen, such that the second subset of the set of icons appears to be rotating consistent with the movement of the gantry.

Clause 9. The computer-readable medium of clauses 1-8.1, wherein the movement parameter of the gantry of the radiotherapy machine comprises any combination of one or more of: a speed of rotation of the movement of the gantry, a direction of rotation of the movement of the gantry, an acceleration of the movement of the gantry, or an angle of rotation of the movement of the gantry.

Clause 10. The computer-readable medium of clauses 1-9, wherein relocating the second subset of the set of icons on the display screen, such that the second subset of the set of icons appear to be rotating consistent with the movement of the gantry further comprises: adjusting the second subset of the set of icons with respect to the display screen at a first angle of rotation opposite of a second angle of rotation of the movement of the gantry.

Clause 11. The computer-readable medium of clauses 1-10, wherein the instructions further cause the one or more processors to: maintain a location of the second subset of the set of icons relative to the display screen such that the second subset of the set of icons rotate about a datum point on the display screen and wherein the second subset of the set of icons are further adjusted at the first angle of rotation about a secondary datum point.

Clause 12. A system comprising: a radiotherapy machine comprising a gantry with a display screen located thereon; one or more processors; and a computer-readable, non-transitory medium with instructions stored therein that when executed by the one or more processors, cause the one or more processors to: present on the display screen located on the gantry of the radiotherapy machine, a set of icons corresponding to one or more attributes of a radiotherapy treatment implemented via the radiotherapy machine; receive a first indication of a movement and/or rotation of the gantry of the radiotherapy machine; and upon receiving the first indication of the movement and/or rotation of the gantry of the radiotherapy machine, relocate and/or reorientate a subset of the set of icons on the display screen, such that the subset of the set of icons appear to be not moving and/or rotating relative to the movement and/or rotation of the gantry of the radiotherapy machine.

Clause 13. The system of clause 12, wherein the instructions further cause the one or more processors to, upon receiving the first indication of the movement and/or rotation of the gantry of the radiotherapy machine, relocate a second subset of the set of icons on the display screen, such that the second subset of the set of icons appear to be rotating consistent with the movement of the gantry.

Clause 14. The system of clauses 12-13, wherein the instructions further cause the one or more processors to: remove the second subset of the set of icons from a first position when the gantry has rotated beyond a first defined position; and display at a second position the second subset of the set of icons previously removed when the gantry has rotated beyond a second defined position.

Clause 14.1. The system of clauses 12-14, wherein the second subset of the set of icons displays an identifier of a patient being treated.

Clause 14.2. The system of clauses 12-14.1 wherein the instructions further cause the one or more processors to disable the display screen when the gantry is at a defined position.

Clause 14.3. The system of clauses 12-14.2, wherein the instructions further cause the one or more processors to obfuscate at least one icon within the set of icons when the gantry reaches a defined position.

Clause 15. The system of clauses 12-14.3, wherein relocating of the second subset of the set of icons corresponds with a movement parameter of the movement of the gantry of the radiotherapy machine.

Clause 16. The system of clauses 12-15, wherein the instructions further cause the one or more processors to: receive a second indication of the movement parameter of the gantry of the radiotherapy machine; determine an icon movement parameter for the second subset of the set of icons, wherein the icon movement parameter corresponds to the movement parameter of the gantry; and upon receiving the first indication of the movement and/or rotation of the gantry of the radiotherapy machine, relocate and/or reorientate the second subset of the set of icons on the display screen in accordance with the icon movement parameter, such that the second subset of the set of icons appear to be rotating with the movement of the gantry while maintaining a first orientation.

Clause 17. The system of clauses 12-16, wherein the instructions further cause the one or more processors to adjust a first orientation of the second subset of the set of icons with respect to the display screen at a first angle of rotation opposite of a second angle of rotation of the movement of the gantry such that a second orientation of the second subset of the set of icons is maintained when relocating the second subset of the set of icons on the display screen, such that the second subset of the set of icons appears to be rotating consistent with the movement of the gantry.

Clause 17.1. The system of clauses 12-17, wherein the movement parameter of the gantry of the radiotherapy machine comprises any combination of one or more of: a speed of rotation of the movement of the gantry, a direction of rotation of the movement of the gantry, an acceleration of the movement of the gantry, or an angle of rotation of the movement of the gantry.

Clause 17.2. The system of clauses 12-17.1, wherein relocating the second subset of the set of icons on the display screen, such that the second subset of the set of icons appear to be rotating consistent with the movement of the gantry further comprises: adjusting the second subset of the set of icons with respect to the display screen at a first angle of rotation opposite of a second angle of rotation of the movement of the gantry.

Clause 18. The system of clauses 12-17.2, wherein the instructions further cause the one or more processors to: maintain a location of the second subset of the set of icons relative to the display screen such that the second subset of the set of icons rotate about a datum point on the display screen and/or wherein the second subset of the set of icons are further adjusted at the first angle of rotation about a secondary datum point.

Clause 19. A method comprising: presenting, by at least one processor on a display screen located on a gantry of a radiotherapy machine, a set of icons corresponding to one or more attributes of a radiotherapy treatment implemented via the radiotherapy machine; receiving, by the at least one processor, a first indication of a movement and/or rotation of the gantry of the radiotherapy machine; and upon receiving the first indication of the movement and/or rotation of the gantry of the radiotherapy machine, relocating and/or reorientating, by the at least one processor, a subset of the set of icons on the display screen, such that the subset of the set of icons appear to be not moving and/or rotating relative to the movement and/or rotation of the gantry of the radiotherapy machine.

Clause 20. The method of clause 19, further comprising: upon receiving the first indication of the movement and/or rotation of the gantry of the radiotherapy machine, relocating, by the at least one processor, a second subset of the set of icons on the display screen, such that the second subset of the set of icons appear to be rotating consistent with the movement of the gantry.

Clause 20.1. The method of clause 19 or clause 20, further comprising: removing the second subset of the set of icons from a first position when the gantry has rotated beyond a first defined position; and displaying at a second position the second subset of the set of icons previously removed when the gantry has rotated beyond a second defined position.

Clause 20.2. The method of any of clause 19 to clause 20.1, wherein the second subset of the set of icons displays an identifier of a patient being treated.

Clause 20.3. The method of any of clause 19 to clause 20.2, further comprising disabling the display screen when the gantry is at a defined position.

Clause 20.4. The method of any of clause 19 to clause 20.3, further comprising obfuscating at least one icon within the set of icons when the gantry reaches a defined position.

Clause 20.5 The method of any of clause 19 to clause 20.4, wherein relocating of the second subset of the set of icons is consistent with a movement parameter of the movement of the gantry of the radiotherapy machine.

Clause 20.6 The method of any of clause 19 to clause 20.5, further comprising receiving a second indication of the movement parameter of the gantry of the radiotherapy machine; determining an icon movement parameter for the second subset of the set of icons, wherein the icon movement parameter corresponds to the movement parameter of the gantry; and upon receiving the first indication of the movement and/or rotation of the gantry of the radiotherapy machine, relocating and/or reorientating the second subset of the set of icons on the display screen in accordance with the icon movement parameter, such that the second subset of the set of icons appear to be rotating with the movement of the gantry, e.g. while maintaining a first orientation.

Clause 20.7. The method of any of clause 19 to clause 20.6, further comprising adjusting a first orientation of the second subset of the set of icons with respect to the display screen at a first angle of rotation opposite of a second angle of rotation of the movement of the gantry such that a second orientation of the second subset of the set of icons is maintained when relocating the second subset of the set of icons on the display screen, such that the second subset of the set of icons appears to be rotating consistent with the movement of the gantry.

Clause 20.8. The method of any of clause 19 to clause 20.7, wherein the movement parameter of the gantry of the radiotherapy machine comprises any combination of one or more of: a speed of rotation of the movement of the gantry, a direction of rotation of the movement of the gantry, an acceleration of the movement of the gantry, or an angle of rotation of the movement of the gantry.

Clause 20.9. The method of any of clause 19 to clause 20.8, wherein relocating the second subset of the set of icons on the display screen, such that the second subset of the set of icons appear to be rotating consistent with the movement of the gantry further comprises: adjusting the second subset of the set of icons with respect to the display screen at a first angle of rotation opposite of a second angle of rotation of the movement of the gantry.

Clause 20.10. The method of any of clause 19 to clause 20.9, further comprising maintaining a location of the second subset of the set of icons relative to the display screen such that the second subset of the set of icons rotate about a datum point on the display screen and wherein the second subset of the set of icons are further adjusted at the first angle of rotation about a secondary datum point.

Clause 21: A computer program comprising instructions which, when the program is executed by a computer, cause the computer to carry out the method of any of clauses 19-20.10.

Clause 22: A computer-readable medium comprising a set of instructions, that when executed by one or more processors, cause the one or more processors to carry out the method of any of clauses 19-20.10.

The presentation of information on the display screen, the user interaction with the display screen and/or the GUI, may have the technical effect of assisting the user in performing a radiotherapy setup workflow by means of a continued and/or guided process of human-machine interaction. The systems discussed herein may be configured to assist the user in performing a radiotherapy workflow and/or a radiotherapy setup workflow, by means of a continued and/or guided process of human-machine interaction.

In this disclosure, the term "radiotherapy treatment" may refer to radiotherapy treatment and/or radiotherapy treatment setup. The method steps may refer to treatment setup, while the systems may be configured to perform treatment and/or treatment setup.

The foregoing method descriptions and the process flow diagrams are provided merely as illustrative examples and are not intended to require or imply that the steps of the various embodiments must be performed in the order presented. The steps in the foregoing embodiments may be performed in any order. Words such as "then," "next," etc. are not intended to limit the order of the steps; these words are simply used to guide the reader through the description of the methods. Although process flow diagrams may describe the operations as a sequential process, many of the operations can be performed in parallel or concurrently. In addition, the order of the operations may be re-arranged. A process may correspond to a method, a function, a procedure, a subroutine, a subprogram, and the like. When a process corresponds to a function, the process termination may correspond to a return of the function to a calling function or a main function.

The various illustrative logical blocks, modules, circuits, and algorithm steps described in connection with the embodiments disclosed herein may be implemented as electronic hardware, computer software, or combinations of both. To clearly illustrate this interchangeability of hardware and software, various illustrative components, blocks, modules, circuits, and steps have been described above generally in terms of their functionality. Whether such functionality is implemented as hardware or software depends upon the particular application and design constraints imposed on the overall system. Skilled artisans may implement the described functionality in varying ways for each particular application, but such implementation decisions should not be interpreted as causing a departure from the scope of the present invention.

Embodiments implemented in computer software may be implemented in software, firmware, middleware, microcode, hardware description languages, or any combination thereof. A code segment or machine-executable instructions may represent a procedure, a function, a subprogram, a program, a routine, a subroutine, a module, a software package, a class, or any combination of instructions, data structures, or program statements. A code segment may be coupled to another code segment or a hardware circuit by passing and/or receiving information, data, arguments, parameters, or memory contents. Information, arguments, parameters, data, etc. may be passed, forwarded, or transmitted via any suitable means including memory sharing, message passing, token passing, network transmission, etc.

The actual software code or specialized control hardware used to implement these systems and methods is not limiting of the invention. Thus, the operation and behavior of the systems and methods were described without reference to the specific software code being understood that software and control hardware can be designed to implement the systems and methods based on the description herein.

When implemented in software, the functions may be stored therein as one or more instructions or code on a computer-readable, non-transitory medium or processor-readable storage medium. The steps of a method or algorithm disclosed herein may be embodied in a processor-executable software module, which may reside on a computer-readable or processor-readable storage medium. A non-transitory computer-readable or processor-readable media includes both computer storage media and tangible storage media that facilitate transfer of a computer program from one place to another. A non-transitory processor-readable storage media may be any available media that may be accessed by a computer. By way of example, and not limitation, such non-transitory processor-readable media may comprise RAM, ROM, EEPROM, CD-ROM, or other optical disk storage, magnetic disk storage or other magnetic storage devices, or any other tangible storage medium that may be used to store desired program code in the form of instructions or data structures and that may be accessed by a computer or processor. Disk and disc, as used herein, include compact disc (CD), laser disc, optical disc, digital versatile disc (DVD), floppy disk, and Blu-ray disc where disks usually reproduce data magnetically, while discs reproduce data optically with lasers. Combinations of the above should also be included within the scope of computer-readable media. Additionally, the operations of a method or algorithm may reside as one or any combination or set of codes and/or instructions on a non-transitory processor-readable medium and/or computer-readable medium, which may be incorporated into a computer program product.

The preceding description of the disclosed embodiments is provided to enable any person skilled in the art to make or use the present invention. Various modifications to these embodiments will be readily apparent to those skilled in the art, and the generic principles defined herein may be applied to other embodiments without departing from the scope of the invention. Thus, the present invention is not intended to be limited to the embodiments shown herein but is to be accorded the widest scope consistent with the following claims and the principles and novel features disclosed herein.

While various aspects and embodiments have been disclosed, other aspects and embodiments are contemplated. The various aspects and embodiments disclosed are for purposes of illustration and are not intended to be limiting with the true scope being indicated by the following claims.

## Claims

1. A computer-readable medium comprising instructions, that when executed by one or more processors, cause the one or more processors to:
present on a display screen located on a gantry of a radiotherapy machine, a set of icons corresponding to one or more attributes of a radiotherapy treatment implemented via the radiotherapy machine;
receive a first indication of a movement of the gantry of the radiotherapy machine; and
upon receiving the first indication of the movement of the gantry of the radiotherapy machine, relocate a subset of the set of icons on the display screen, such that the subset of the set of icons appears to be not rotating relative to a movement of the gantry of the radiotherapy machine.

2. The computer-readable medium of claim 1, wherein the instructions further cause the one or more processors to, upon receiving the first indication of the movement of the gantry of the radiotherapy machine, relocate a second subset of the set of icons on the display screen, such that the second subset of the set of icons appear to be rotating consistent with the movement of the gantry.

3. The computer-readable medium of claim 2, wherein the instructions further cause the one or more processors to:
remove the second subset of the set of icons from a first position when the gantry has rotated beyond a first defined position; and
display at a second position the second subset of the set of icons previously removed when the gantry has rotated beyond a second defined position.

4. The computer-readable medium of claim 2 or claim 3, wherein the second subset of the set of icons displays an identifier of a patient being treated.

5. The computer-readable medium of any preceding claim, wherein the instructions further cause the one or more processors to :
disabling the display screen when the gantry is at a defined position;
and/or:
wherein the instructions further cause the one or more processors to:
obfuscate at least one icon within the set of icons when the gantry reaches a defined position.

6. The computer-readable medium of any of claim 2 to claim 4, wherein relocating of the second subset of the set of icons is consistent with a movement parameter of the movement of the gantry of the radiotherapy machine.

7. The computer-readable medium of claim 6, wherein the instructions further cause the one or more processors to:
receive a second indication of the movement parameter of the gantry of the radiotherapy machine;
determine an icon movement parameter for the second subset of the set of icons, wherein the icon movement parameter corresponds to the movement parameter of the gantry; and
upon receiving the first indication of the movement of the gantry of the radiotherapy machine, relocate the second subset of the set of icons on the display screen in accordance with the icon movement parameter, such that the second subset of the set of icons appear to be rotating with the movement of the gantry;
and optionally:
wherein the movement parameter of the gantry of the radiotherapy machine comprises at least one of a speed of rotation of the movement of the gantry, a direction of rotation of the movement of the gantry, an acceleration of the movement of the gantry, or an angle of rotation of the movement of the gantry.

8. The computer-readable medium of any of claim 2 to claim 4, wherein relocating the second subset of the set of icons on the display screen, such that the second subset of the set of icons appear to be rotating consistent with the movement of the gantry further comprises:
adjusting the second subset of the set of icons with respect to the display screen at a first angle of rotation opposite of a second angle of rotation of the movement of the gantry;
and optionally:
wherein the instructions further cause the one or more processors to:
maintain a location of the second subset of the set of icons relative to the display screen such that the second subset of the set of icons rotate about a datum point on the display screen and wherein the second subset of the set of icons are further adjusted at the first angle of rotation about a secondary datum point.

9. A system comprising:
a radiotherapy machine comprising a gantry with a display screen located thereon;
one or more processors; and
a computer-readable, non-transitory medium with instructions stored therein that when executed by the one or more processors, cause the one or more processors to:
present on the display screen located on the gantry of the radiotherapy machine, a set of icons corresponding to one or more attributes of a radiotherapy treatment implemented via the radiotherapy machine;
receive a first indication of a movement of the gantry of the radiotherapy machine; and
upon receiving the first indication of the movement of the gantry of the radiotherapy machine, relocate a subset of the set of icons on the display screen, such that the subset of the set of icons appear to be not rotating relative to the movement of the gantry of the radiotherapy machine.

10. The system of claim 9, wherein the instructions further cause the one or more processors to, upon receiving the first indication of the movement of the gantry of the radiotherapy machine, relocate a second subset of the set of icons on the display screen, such that the second subset of the set of icons appear to be rotating consistent with the movement of the gantry.

11. The system of claim 10, wherein the instructions further cause the one or more processors to:
remove the second subset of the set of icons from a first position when the gantry has rotated beyond a first defined position; and
display at a second position the second subset of the set of icons previously removed when the gantry has rotated beyond a second defined position.

12. The system of claim 10 or claim 11, wherein relocating of the second subset of the set of icons corresponds with a movement parameter of the movement of the gantry of the radiotherapy machine;
and optionally:
wherein the instructions further cause the one or more processors to:
receive a second indication of the movement parameter of the gantry of the radiotherapy machine;
determine an icon movement parameter for the second subset of the set of icons, wherein the icon movement parameter corresponds to the movement parameter of the gantry; and
upon receiving the first indication of the movement of the gantry of the radiotherapy machine, relocate the second subset of the set of icons on the display screen in accordance with the icon movement parameter, such that the second subset of the set of icons appear to be rotating with the movement of the gantry while maintaining a first orientation.

13. The system of any of claim 10 to claim 12, wherein the instructions further cause the one or more processors to adjust a first orientation of the second subset of the set of icons with respect to the display screen at a first angle of rotation opposite of a second angle of rotation of the movement of the gantry such that a second orientation of the second subset of the set of icons is maintained when relocating the second subset of the set of icons on the display screen, such that the second subset of the set of icons appears to be rotating consistent with the movement of the gantry;
and optionally:
wherein the instructions further cause the one or more processors to maintain a location of the second subset of the set of icons relative to the display screen such that the second subset of the set of icons rotate about a datum point on the display screen.

14. A method comprising:
presenting, by at least one processor on a display screen located on a gantry of a radiotherapy machine, a set of icons corresponding to one or more attributes of a radiotherapy treatment implemented via the radiotherapy machine;
receiving, by the at least one processor, a first indication of a movement of the gantry of the radiotherapy machine; and
upon receiving the first indication of the movement of the gantry of the radiotherapy machine, relocating, by the at least one processor, a subset of the set of icons on the display screen, such that the subset of the set of icons appear to be not rotating relative to the movement of the gantry of the radiotherapy machine.

15. The method of claim 14, further comprising:
upon receiving the first indication of the movement of the gantry of the radiotherapy machine, relocating, by the at least one processor, a second subset of the set of icons on the display screen, such that the second subset of the set of icons appear to be rotating consistent with the movement of the gantry.
